# EUROPEAN PATENT APPLICATION

(11) **EP 4 832 009 A1**
(43) Date of publication of application: **28.10.2026**
(21) Application number: 26173794.4
(22) Date of filing: 21.04.2026
(51) Int. Cl.: G16H 50/20, A61B 8/00, A61B 8/06, G16H 50/30, A61B 8/08, G06T 7/00

(54) **ULTRASONIC DIAGNOSTIC APPARATUS, MEDICAL IMAGE PROCESSING APPARATUS, MEDICAL IMAGE PROCESSING METHOD AND PROGRAM**

(30) Priority: 21.04.2025 JP 2025069944
(71) Applicant: Canon Kabushiki Kaisha, Tokyo 146-8501 (JP)
(72) Inventor: TANIMURA, Shun, Sapporo City (JP); NARITA, Akihiro, Sapporo City (JP); HENMI, Mihoko, Sapporo City (JP); WATANABE, Masaki, Tokyo (JP); SONE, Yukari, Tokyo (JP); MIYAKI, Keisuke, Tokyo (JP); TAKAHASHI, Mikinori, Tokyo (JP)
(74) Representative: Marks & Clerk LLP

(57) **Abstract**

An ultrasonic diagnostic apparatus according to an embodiment includes an acquisition unit, a first evaluation information acquisition unit, a second evaluation information acquisition unit, an index value calculation unit, and a display control unit. The acquisition unit acquires ultrasonic image data indicating a target part of a subject. The first evaluation information acquisition unit acquires first evaluation information for evaluating a state of rheumatism in the target part indicated by the ultrasonic image data. The second evaluation information acquisition unit acquires second evaluation information for evaluating the state of rheumatism in the target part. The index value calculation unit calculates an index value indicating the state of rheumatism based on information including at least the first evaluation information and the second evaluation information. The display control unit displays information capable of identifying a category corresponding to the index value among a plurality of categories indicating a degree of the state of rheumatism.

## Description

### CROSS REFERENCE TO RELATED APPLICATIONS

This application is based upon and claims the benefit of priority from the prior Japanese Patent Application No. 2025-069944, filed on April 21, 2025, the entire contents of which are incorporated herein by reference.

### FIELD

Embodiments described in the present specification and drawings relate to an ultrasonic diagnostic apparatus, a medical image processing apparatus, a medical image processing method and a program.

### BACKGROUND

In related art, an ultrasonic diagnostic apparatus has been used for diagnosis of rheumatism. When rheumatism is diagnosed by using this ultrasonic diagnostic apparatus, it is required by guidelines for diagnosing rheumatism to score (grade) based on a blood flow signal in a synovial region indicated by ultrasonic image data.

However, since this scoring may be performed visually by an inspector, there is a high degree of dependence on the inspector. In order to accurately grasp a state of rheumatism of a patient who is a subject, it may be desired to evaluate the state of rheumatism with a finer level of granularity than four grades including grade 0, grade 1, grade 2, and grade 3, which are required in the guideline. Thus, it is desired that the state of rheumatism can be evaluated with a fine level of granularity and the degree of dependence on the inspector in the diagnosis of rheumatism is reduced.

### SUMMARY OF INVENTION

An ultrasonic diagnostic apparatus comprising
processing circuitry configured to:
acquire ultrasonic image data indicating a target part of a subject;
acquire first evaluation information for evaluating a state of rheumatism in the target part indicated by the ultrasonic image data;
acquire second evaluation information for evaluating the state of rheumatism in the target part;
calculate an index value indicating the state of rheumatism based on information including at least the first evaluation information and the second evaluation information; and
display information capable of identifying a category corresponding to the index value among a plurality of categories indicating a degree of the state of rheumatism.

pieces of visual information different from each other may be assigned to the plurality of categories.

The first evaluation information may be a synovial region.

The visual information may be given to at least a part of the synovial region.

The processing circuitry may be configured to detect the synovial region in each of a plurality of pieces of processed image data by inputting, to a trained model, each of the plurality of pieces of processed image data from each other obtained by executing image processing on the ultrasonic image data, the trained model outputting the synovial region in the ultrasonic image data by inputting the ultrasonic image data.

An imaging order may be set for a plurality of target parts of the subject.

The processing circuitry may be configured to register the information capable of identifying the category for each of the plurality of target parts.

The processing circuitry may be configured to register preset information including the imaging order as a protocol.

The processing circuitry may be configured to display information based on the imaging order during execution of the protocol.

The processing circuitry may be configured to select a body mark corresponding to the imaging order based on the preset information.

The processing circuitry may be configured to display information regarding a target part of the subject to be imaged next, as the information based on the imaging order.

The processing circuitry may be configured to display information indicating that imaging is completed, as the information based on the imaging order.

The processing circuitry may be further configured to calculate, as the index value, a ratio of the number of pixels of the blood flow signal in the synovial region to the number of pixels of the synovial region based on the number of pixels of the synovial region and the number of pixels of the blood flow signal in the synovial region.

A medical image processing apparatus comprising
processing circuitry configured to:
acquire ultrasonic image data indicating a target part of a subject;
acquire first evaluation information for evaluating a state of rheumatism in the target part indicated by the ultrasonic image data;
acquire second evaluation information for evaluating the state of rheumatism in the target part;
calculate an index value indicating the state of rheumatism based on information including at least the first evaluation information and the second evaluation information; and
display information capable of identifying a category corresponding to the index value among a plurality of categories indicating a degree of the state of rheumatism.

A medical image processing method comprising:
acquiring ultrasonic image data indicating a target part of a subject;
acquiring first evaluation information for evaluating a state of rheumatism in the target part indicated by the ultrasonic image data;
acquiring second evaluation information for evaluating the state of rheumatism in the target part;
calculating an index value indicating the state of rheumatism based on information including at least the first evaluation information and the second evaluation information; and
displaying information capable of identifying a category corresponding to the index value among a plurality of categories indicating a degree of the state of rheumatism.

A program causing a computer to:
acquire ultrasonic image data indicating a target part of a subject;
acquire first evaluation information for evaluating a state of rheumatism in the target part indicated by the ultrasonic image data;
acquire second evaluation information for evaluating the state of rheumatism in the target part;
calculate an index value indicating the state of rheumatism based on information including at least the first evaluation information and the second evaluation information; and
display information capable of identifying a category corresponding to the index value among a plurality of categories indicating a degree of the state of rheumatism.

### BRIEF DESCRIPTION OF THE DRAWINGS

FIG. 1 is a block diagram illustrating an example of a configuration of an ultrasonic diagnostic apparatus according to a first embodiment.
FIG. 2 is a diagram illustrating an example of a preset information setting screen according to the first embodiment.
FIG. 3 is a diagram illustrating an example of a user setting screen;
FIG. 4 is an explanatory diagram for describing an example of a data flow at the time of learning of a detection function.
FIG. 5 is an explanatory diagram for describing an example of a data flow at the time of operation of the detection function.
FIG. 6 is a flowchart for describing screen display processing executed in the ultrasonic diagnostic apparatus according to the first embodiment.
FIG. 7 is a diagram illustrating an example of a preset information selection screen.
FIG. 8 is a diagram illustrating an example of an inspection execution screen.
FIG. 9 is a diagram illustrating an example of the inspection execution screen including information indicating that imaging is completed.
FIG. 10 is a diagram illustrating an example of a result display screen according to the first embodiment.
FIG. 11 is a diagram illustrating an example of a result display screen when a predetermined operation is performed on the result display screen illustrated in FIG. 10.
FIG. 12 is a flowchart for describing detection processing executed in the ultrasonic diagnostic apparatus according to the first embodiment.
FIG. 13 is a diagram illustrating an example of ultrasonic image data according to the first embodiment.
FIG. 14A is a diagram illustrating an example of a plurality of pieces of processed image data according to the first embodiment.
FIG. 14B is a diagram illustrating an example of the plurality of pieces of processed image data according to the first embodiment.
FIG. 14C is a diagram illustrating an example of the plurality of pieces of processed image data according to the first embodiment.
FIG. 15A is a diagram illustrating an example of a synovial region detected in each of the plurality of pieces of processed image data according to the first embodiment.
FIG. 15B is a diagram illustrating an example of the synovial region detected in each of the plurality of pieces of processed image data according to the first embodiment.
FIG. 15C is a diagram illustrating an example of the synovial region detected in each of the plurality of pieces of processed image data according to the first embodiment.
FIG. 16 is a diagram illustrating an example of a detection result display screen.
FIG. 17 is a flowchart for describing index value calculation processing executed in the ultrasonic diagnostic apparatus according to the first embodiment.
FIG. 18 is a diagram illustrating an example of the ultrasonic image data and the synovial region.
FIG. 19 is a diagram illustrating an example of a calculation result of an index value in the ultrasonic image data illustrated in FIG. 18.
FIG. 20 is a diagram illustrating an example of information capable of identifying a category corresponding to the index value illustrated in FIG. 19.
FIG. 21 is a diagram illustrating an example of a result display screen according to Modification 2.
FIG. 22 is a diagram illustrating an example of a result display screen according to Modification 3.
FIG. 23 is a diagram illustrating an example of a result display screen according to Modification 4.
FIG. 24 is a diagram illustrating an example of a result display screen according to Modification 5.
FIG. 25 is a diagram illustrating an example of a result display screen according to Modification 6.
FIG. 26 is a flowchart for describing index value calculation processing according to Modification 7.
FIG. 27A is a diagram illustrating an example of first evaluation information and second evaluation information according to Modification 7.
FIG. 27B is a diagram illustrating an example of the first evaluation information and the second evaluation information according to Modification 7.
FIG. 28 is a block diagram illustrating an example of a medical image processing system according to a second embodiment.
FIG. 29 is a diagram illustrating an example of an optical image.
FIG. 30 is a diagram illustrating an example of a preset information setting screen.

### DETAILED DESCRIPTION

Hereinafter, respective embodiments of the ultrasonic diagnostic apparatus, the medical image processing apparatus, a medical image processing method and a program will be described with reference to the accompanying drawings. In the embodiments below, the same reference signs are given for identical components in terms of configuration and function, and duplicate description is omitted.

### [First Embodiment]

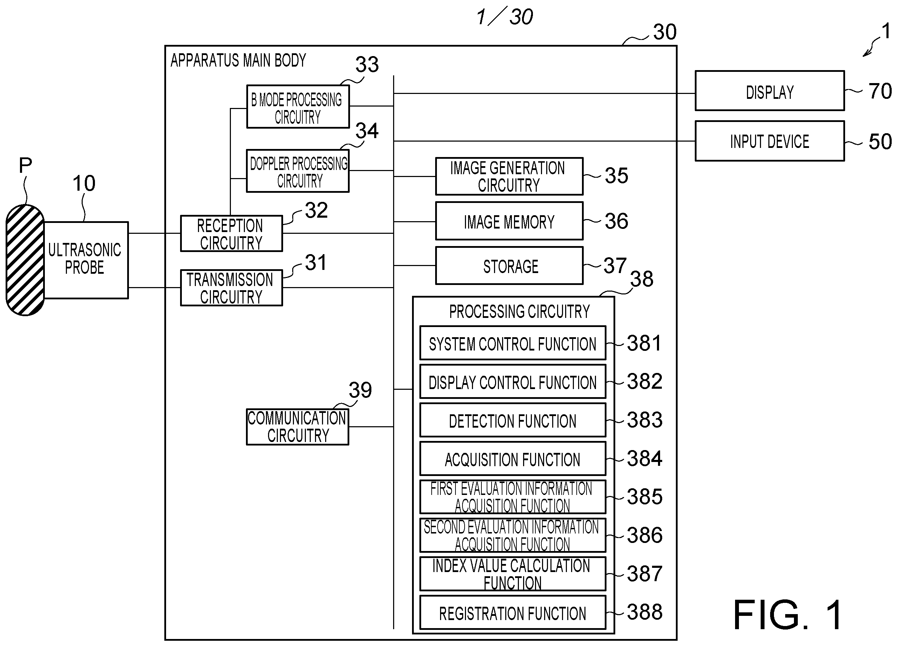

FIG. 1 is a block diagram illustrating an example of a configuration of an ultrasonic diagnostic apparatus according to a first embodiment. As illustrated in FIG. 1, an ultrasonic diagnostic apparatus 1 includes an ultrasonic probe 10, an apparatus main body 30, an input device 50, and a display 70.

The ultrasonic probe 10 includes, for example, a plurality of piezoelectric vibrators. The plurality of piezoelectric vibrators generate ultrasonic waves based on a drive signal supplied from transmission circuitry 31 included in the apparatus main body 30 to be described later. The ultrasonic probe 10 receives a reflected wave from a subject P and converts the reflected wave into an electric signal. The ultrasonic probe 10 includes, for example, a matching layer provided on the piezoelectric vibrator, a backing material for preventing propagation of the ultrasonic waves rearward from the piezoelectric vibrator and the like.

When the ultrasonic waves are transmitted from the ultrasonic probe 10 to the subject P, the transmitted ultrasonic waves are reflected one after another on a discontinuous surface of an acoustic impedance in a body tissue of the subject P, and are received as a reflected wave signal by the plurality of piezoelectric vibrators included in the ultrasonic probe 10. An amplitude of the received reflected wave signal depends on a difference in acoustic impedance at the discontinuous surface at which the ultrasonic waves are reflected. The reflected wave signal when transmitted ultrasonic pulses are reflected by a moving blood flow or a surface of a heart wall or the like receives a frequency shift depending on a velocity component of a moving body with respect to a transmission direction of an ultrasonic wave, due to the Doppler effect.

The ultrasonic probe 10 is detachably connected to the apparatus main body 30. When scanning of a two-dimensional region in the subject P (two-dimensional scanning) is performed, a user connects, for example, a 1D array probe in which a plurality of piezoelectric vibrators are arranged in a line to the apparatus main body 30, as the ultrasonic probe 10. The 1D array probe is a linear type ultrasonic probe, a convex type ultrasonic probe, a sector type ultrasonic probe, or the like. When scanning of a three-dimensional region in the subject P (three-dimensional scanning) is performed, the user connects, for example, a mechanical 4D probe or a 2D array probe to the apparatus main body 30, as the ultrasonic probe 10. The mechanical 4D probe can perform two-dimensional scanning by using a plurality of piezoelectric vibrators arranged in a line like the 1D array probe, and can perform three-dimensional scanning by swinging the plurality of piezoelectric vibrators at a predetermined angle (swing angle). The 2D array probe can perform three-dimensional scanning by a plurality of piezoelectric vibrators arranged in a matrix, and can perform two-dimensional scanning by focusing and transmitting ultrasonic waves. The 2D array probe can simultaneously perform two-dimensional scanning of a plurality of cross sections.

The apparatus main body 30 generates ultrasonic image data based on a signal from the ultrasonic probe 10. Specifically, the apparatus main body 30 can generate two-dimensional ultrasonic image data based on the reflected wave signal corresponding to the two-dimensional region of the subject P received by the ultrasonic probe 10. The apparatus main body 30 can generate three-dimensional ultrasonic image data based on the reflected wave signal corresponding to the three-dimensional region of the subject P received by the ultrasonic probe 10. As illustrated in FIG. 1, the apparatus main body 30 includes transmission circuitry 31, reception circuitry 32, B mode processing circuitry 33, Doppler processing circuitry 34, image generation circuitry 35, image memory 36, storage 37, processing circuitry 38, and communication circuitry 39.

The transmission circuitry 31 includes a pulse generator, a transmission delay unit, a pulser, and the like, and supplies a drive signal to the ultrasonic probe 10. The pulse generator repeatedly generates rate pulses for forming transmission ultrasonic waves at a predetermined rate frequency. The transmission delay unit focuses the ultrasonic waves generated from the ultrasonic probe 10 into a beam shape, and gives, to each rate pulse generated by the pulse generator, a delay time for each piezoelectric vibrator necessary to determine transmission directivity. The pulser applies the drive signal (drive pulse) to the ultrasonic probe 10 at a timing based on the rate pulse. That is, the transmission delay unit arbitrarily adjusts a transmission direction of an ultrasonic wave transmitted from a piezoelectric vibrator surface by changing the delay time given to each rate pulse.

The transmission circuitry 31 has a function of instantaneously changing a transmission frequency, a transmission driving voltage, and the like in order to execute a predetermined scan sequence based on an instruction from the processing circuitry 38 to be described later. In particular, the transmission driving voltage is changed by linear amplifier type transmission circuitry capable of instantaneously switching a value thereof or a mechanism for electrically switching a plurality of power supply units.

The reception circuitry 32 includes a preamplifier, an analog/digital (A/D) converter, a reception delay unit, an adder, and the like, and performs various types of processing on the reflected wave signal received by the ultrasonic probe 10 to generate reflected wave data. The preamplifier amplifies the reflected wave signal for each channel and performs gain adjustment (gain correction). The A/D converter converts the gain-corrected reflected wave signal into a digital signal by A/D converting the gain-corrected reflected wave signal. The reception delay unit gives a delay time necessary to determine reception directivity. The adder performs processing of adding the reflected wave signal processed by the reception delay unit, and generates the reflected wave data. The adder outputs the generated reflected wave data to the B mode processing circuitry 33 and the Doppler processing circuitry 34.

When the subject P is two-dimensionally scanned, the transmission circuitry 31 causes the ultrasonic probe 10 to transmit a two-dimensional ultrasonic beam. The reception circuitry 32 generates two-dimensional reflected wave data from a two-dimensional reflected wave signal received by the ultrasonic probe 10. When the subject P is three-dimensionally scanned, the transmission circuitry 31 according to the present embodiment causes the ultrasonic probe 10 to transmit a three-dimensional ultrasonic beam. The reception circuitry 32 generates three-dimensional reflected wave data from a three-dimensional reflected wave signal received by the ultrasonic probe 10.

The B mode processing circuitry 33 receives the reflected wave data from the reception circuitry 32, performs logarithmic amplification, envelope detection processing, and the like, and generates data (B mode data) in which signal intensity is expressed by brightness of luminance.

The Doppler processing circuitry 34 performs frequency analysis on velocity information from the reflected wave data received from the reception circuitry 32, extracts a blood flow, a tissue, and a contrast medium echo component by the Doppler effect, and generates data (Doppler data) obtained by extracting moving body information such as velocity, variance, and power for multiple points. Here, the moving body is, for example, a blood flow, a tissue of an organ that periodically moves such as a heart wall, or a contrast medium.

The B mode processing circuitry 33 and the Doppler processing circuitry 34 can process both types of two-dimensional reflected wave data and three-dimensional reflected wave data. That is, the B mode processing circuitry 33 generates two-dimensional B mode data from the two-dimensional reflected wave data, and generates three-dimensional B mode data from the three-dimensional reflected wave data. The Doppler processing circuitry 34 generates two-dimensional Doppler data from the two-dimensional reflected wave data and generates three-dimensional Doppler data from the three-dimensional reflected wave data.

The image generation circuitry 35 generates ultrasonic image data expressed in a predetermined luminance range based on the signal received by the ultrasonic probe 10. For example, the image generation circuitry 35 generates, as the ultrasonic image data, two-dimensional B mode image data in which intensity of the reflected wave is represented by luminance from the two-dimensional B mode data generated by the B mode processing circuitry 33. The image generation circuitry 35 generates, as the ultrasonic image data, two-dimensional color Doppler image data as average speed image data, variance image data, power image data, or combination image data thereof, indicating the moving body information from the two-dimensional Doppler data generated by the Doppler processing circuitry 34.

Here, the image generation circuitry 35 generally converts (scan-converts) a scanning line signal sequence of ultrasonic scanning into a scanning line signal sequence of a video format indicated by a television or the like, and generates display ultrasonic image data. For example, the image generation circuitry 35 generates the display ultrasonic image data by performing coordinate conversion according to a scanning mode of the ultrasonic wave by the ultrasonic probe 10. For example, the image generation circuitry 35 performs, as various types of image processing other than the scan conversion, image processing (smoothing processing) of regenerating an average value image of luminance by using a plurality of image frames after scan conversion, image processing (edge enhancement processing) using a differential filter in the image, and the like.

That is, the B mode data and the Doppler data are data before scan conversion processing, and the data generated by the image generation circuitry 35 is display ultrasonic image data after the scan conversion processing. The B mode data and the Doppler data are also referred to as raw data. The image generation circuitry 35 generates the two-dimensional B mode image data or the two-dimensional color Doppler image data, which is the two-dimensional ultrasonic image data, from the two-dimensional B mode data or the two-dimensional Doppler data, which is the raw data. The image generation circuitry 35 can also generate, for example, a superimposed image in which the color Doppler image data is superimposed on the two-dimensional B mode image data.

For example, the image generation circuitry 35 generates M mode image data from time-series data of the B mode data on one scanning line generated by the B mode processing circuitry 33. The image generation circuitry 35 generates a Doppler waveform obtained by plotting velocity information of a blood flow and tissue in time series from the Doppler data generated by the Doppler processing circuitry 34.

The image generation circuitry 35 can also generate three-dimensional B mode image data by performing coordinate conversion on the three-dimensional B mode data generated by the B mode processing circuitry 33. The image generation circuitry 35 can also generate three-dimensional color Doppler image data by performing coordinate conversion on the three-dimensional Doppler data generated by the Doppler processing circuitry 34.

The image memory 36 is a memory that stores various types of image data generated by the image generation circuitry 35. The image memory 36 also stores pieces of data generated by the B mode processing circuitry 33 and the Doppler processing circuitry 34. The B mode data and the Doppler data stored in the image memory 36 can be called by an operator after diagnosis, for example, and become display ultrasonic image data via the image generation circuitry 35. The image memory 36 also stores the reflected wave data output from the reception circuitry 32. For example, the image memory 36 is realized by a random access memory (RAM), a semiconductor memory element such as a flash memory, a hard disk, or an optical disk.

The storage 37 stores control programs for performing ultrasonic wave transmission and reception, image processing, and display processing, and various types of data such as protocols and various body marks. The storage 37 is also used to store pieces of data stored in the image memory 36, as necessary. For example, the storage 37 is realized by a semiconductor memory element such as a flash memory, a hard disk, or an optical disk.

The processing circuitry 38 executes various types of data processing. The processing circuitry 38 has a system control function 381, a display control function 382, a detection function 383, an acquisition function 384, a first evaluation information acquisition function 385, a second evaluation information acquisition function 386, an index value calculation function 387, and a registration function 388. Here, for example, the processing functions of the system control function 381, the display control function 382, the detection function 383, the acquisition function 384, the first evaluation information acquisition function 385, the second evaluation information acquisition function 386, the index value calculation function 387, and the registration function 388, which are components of the processing circuitry 38 illustrated in FIG. 1, are recorded in the storage 37 in the form of programs executable by a computer. The processing circuitry 38 realizes a function corresponding to each program by reading each program from the storage 37 and executing each read program. In other words, the processing circuitry 38 in a state of reading each program has the functions of the processing circuitry 38 illustrated in FIG. 1. The processing circuitry 38 is realized by, for example, a processor. The system control function 381 is an example of a control unit. The display control function 382 is an example of a display control unit. The detection function 383 is an example of a detection unit. The acquisition function 384 is an example of an acquisition unit. The first evaluation information acquisition function 385 is an example of a first evaluation information acquisition unit. The second evaluation information acquisition function 386 is an example of a second evaluation information acquisition unit. The index value calculation function 387 is an example of an index value calculation unit. The registration function 388 is an example of a registration unit.

The system control function 381 is a function of integrally controlling the entire operation of the ultrasonic diagnostic apparatus 1. For example, the system control function 381 controls the transmission circuitry 31 and the reception circuitry 32 based on parameters related to transmission and reception of ultrasonic waves corresponding to various modes. The various modes include, for example, a B mode, a Doppler mode, and the like.

The display control function 382 displays information capable of identifying a category corresponding to the index value, among a plurality of categories indicating a degree of a state of rheumatism. Hereinafter, the category is also referred to as a grade or a score. Pieces of visual information different from each other are assigned to the plurality of categories. The visual information is, for example, color, brightness, saturation, pattern, and the like. The attached figures are provided in grayscale. However, in practice, the display could be a color display. For example, in the present embodiment, the plurality of categories includes six categories. Specifically, the plurality of categories includes a first category indicating grade 0, a second category indicating grade 1, a third category indicating grade 2-, a fourth category indicating grade 2, a fifth category indicating grade 2+, and a sixth category indicating grade 3. In the present embodiment, no color is assigned as the visual information to the first category, green is assigned as the visual information to the second category, yellowish green is assigned as the visual information to the third category, yellow is assigned as the visual information to the fourth category, orange is assigned as the visual information to the fifth category, and red is assigned as the visual information to the sixth category.

The index value is a value indicating a state of rheumatism. The index value is, for example, a ratio of the number of pixels of a blood flow signal in a synovial region to the number of pixels of the synovial region, the number of pixels of the blood flow signal in the synovial region, the number of pixels of the blood flow signal in the BOX, a ratio of the number of pixels of the blood flow signal in the BOX to the number of pixels in the BOX, a ratio of the number of pixels of the synovial region beyond a straight line of a bone surface to the number of pixels of the synovial region not beyond the straight line of the bone surface, the number of pixels of the synovial region beyond the straight line of the bone surface/the number of pixels of the entire synovial region, or the like. The ratio of the number of pixels of the blood flow signal in the synovial region to the number of pixels of the synovial region and the ratio of the number of pixels of the blood flow signal in the BOX to the number of pixels in the BOX are expressed as Vascularity values.

The display control function 382 displays a preset information setting screen for setting preset information and a user setting screen for setting user setting information. The display control function 382 registers information capable of identifying a category for each of the plurality of target parts. The target part referred to herein is a part at which rheumatism is inspected and diagnosed, and is mainly various joints such as fingers of hands and feet, arms, and ankles, but also includes joint external parts such as eyes, lungs, hearts, digestive organs, and urinary organs (since symptoms of rheumatism may also appear in the joint external parts). Hereinafter, an example in which the target part is the joint of the finger will be described.

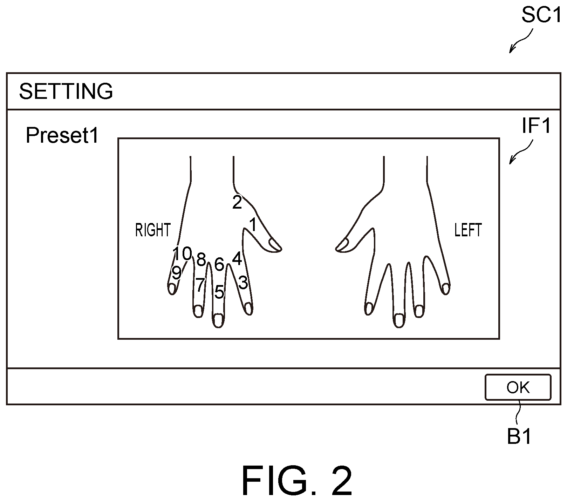

FIG. 2 is a diagram illustrating an example of the preset information setting screen according to the first embodiment. As illustrated in FIG. 2, preset information IF1 and a first button B1 are displayed on a preset information setting screen SC1. The preset information IF1 includes an imaging order and information based on the imaging order. Specifically, the preset information IF1 includes an imaging order for a plurality of joint parts of the subject P and information based on the imaging order. That is, the imaging order is set for each of the plurality of joint parts of the subject P. The information based on the imaging order includes, for example, a joint name. In the example illustrated in FIG. 2, in the preset information IF1, the imaging order for the joint part of the right hand among the plurality of joint parts is set.

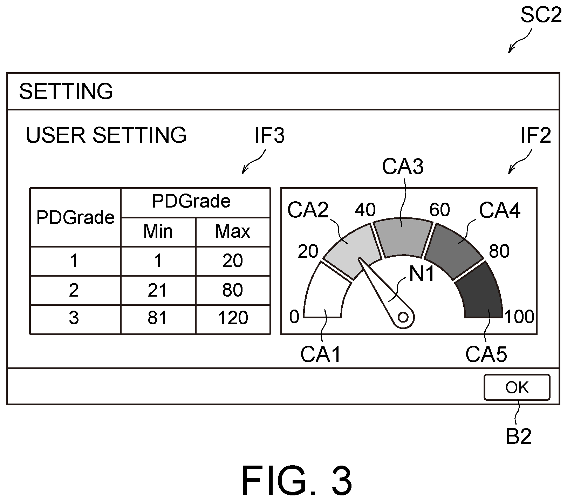

FIG. 3 is a diagram illustrating an example of the user setting screen. As illustrated in FIG. 3, information IF2 capable of identifying the category corresponding to the index value among the plurality of categories indicating the degree of the state of rheumatism, and threshold information IF3 are displayed on a user setting screen SC2. In the example illustrated in FIG. 3, the information IF2 capable of identifying the category corresponding to the index value is an indicator. The indicator includes a plurality of graduations corresponding to each of the plurality of categories and an indicator needle N1.

The plurality of graduations is graduations indicating the plurality of categories. In the example illustrated in FIG. 3, the number of categories indicating the degree of the state of rheumatism is six, and the first category indicating grade 0 shows only when the index value is 0. Thus, the plurality of graduations includes five graduations CA1 to CA5 indicating categories other than the first category. The pieces of visual information assigned to the plurality of categories are assigned to the five graduations CA1 to CA5. In the example illustrated in FIG. 3, green assigned to the second category is assigned to the first graduation CA1, yellowish green assigned to the third category is assigned to the second graduation CA2, yellow assigned to the fourth category is assigned to the third graduation CA3, orange assigned to the fifth category is assigned to the fourth graduation CA4, and red assigned to the sixth category is assigned to the fifth graduation CA5.

The indicator needle N1 is a needle for indicating the category corresponding to the index value. In the example illustrated in FIG. 3, since the index value corresponds to the third category, that is, grade 2, the indicator needle N1 indicates the third category.

The threshold information IF3 is information regarding a threshold range of the index value set for each category. In the example illustrated in FIG. 3, in the threshold information IF3, a threshold range of the Vascularity value is associated with PDGrade, which is a plurality of categories indicating the degree of the state of rheumatism. In the example illustrated in FIG. 3, when the Vascularity value is 0 as the threshold range of the Vascularity value, the category corresponding to the index value is identified as the first category corresponding to grade 0. When the Vascularity value is 1 or more and 20 or less as the threshold range of the Vascularity value, the category corresponding to the index value is identified as the second category corresponding to grade 1. When the Vascularity value is 21 or more and 80 or less as the threshold range of the Vascularity value, the category corresponding to the index value is identified as one of the third to fifth categories corresponding to grade 2-, grade 2, and grade 2+. Specifically, in the example illustrated in FIG. 3, the threshold range having the Vascularity value of 21 or more and 80 or less is divided into three equal subranges. When the Vascularity value is 21 or more and 40 or less, the category corresponding to the index value is identified as 2-, when the Vascularity value is 41 or more and 60 or less, the category corresponding to the index value is identified as 2, and when the Vascularity value is 61 or more and 80 or less, the category corresponding to the index value is identified as 2+. When the Vascularity value is 81 or more and 100 or less as the threshold range of the Vascularity value, the category corresponding to the index value is identified as the sixth category corresponding to grade 3.

The display control function 382 displays information based on the imaging order during execution of the protocol. The information based on the imaging order may include, for example, information regarding a joint part to be imaged next or information indicating that imaging is completed, together with the joint name. The information regarding the joint part to be imaged next is, for example, information such as a next joint part name and/or a figure for indicating the next joint part. The information indicating that imaging is completed is a check mark superimposed on information schematically indicating the joint part of the subject, or visual information assigned to the joint part for which imaging is completed in the information schematically indicating the joint part of the subject. The visual information assigned to the joint part for which imaging is completed is, for example, a color or a pattern applied to the part for which imaging is completed.

The detection function 383 detects a synovial region in each of a plurality of pieces of processed image data by inputting, to a trained model, each of the plurality of pieces of processed image data different from each other obtained by executing image processing on the ultrasonic image data. The trained model outputs the synovial region in the ultrasonic image data by inputting the ultrasonic image data.

The trained model is a machine-trained model so as to output the synovial region in the ultrasonic image data by inputting the ultrasonic image data. For example, machine learning using a support vector machine (SVM) or deep learning using a multilayer neural network such as a convolutional neural network (CNN) or a convolutional deep belief neural network may be used as the machine learning. In the following description, an example in which the machine learning is deep learning using a neural network and the machine learning model is a trained model using deep learning will be described.

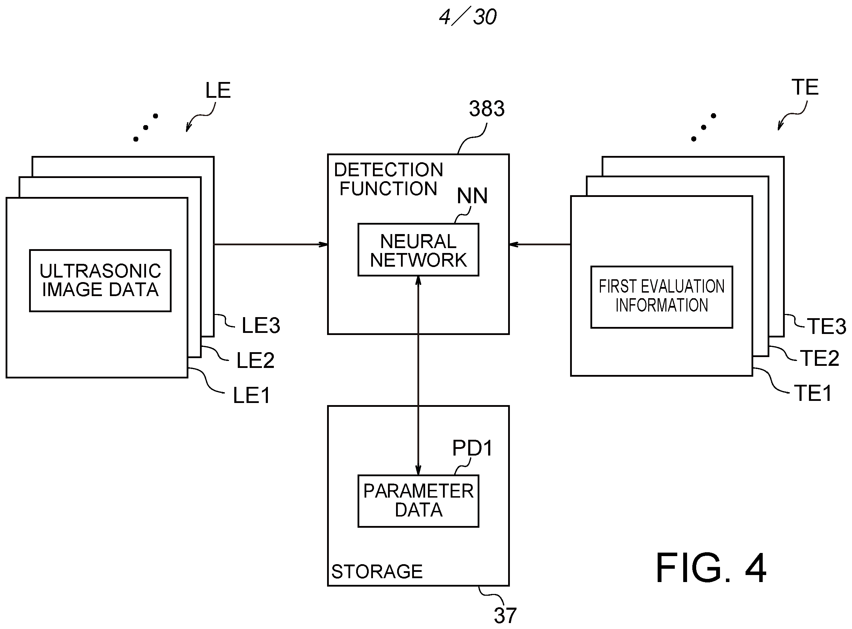

FIG. 4 is an explanatory diagram for describing an example of a data flow at the time of learning of the detection function 383. As illustrated in FIG. 4, at the time of learning, the detection function 383 sequentially updates parameter data PD1 stored in the storage 37 by performing deep learning using a large number of training data sets.

Each training data set of the large number of training data sets includes a set of a learning data group LE and a supervisory data group TE. The learning data group LE includes pieces of learning ultrasonic image data LE1, LE2, LE3, ···. The supervisory data group TE corresponding to each learning ultrasonic image data includes pieces of first evaluation information TE1, TE2, TE3, ..., and the pieces of first evaluation information TE1, TE2, TE3, ··· of each supervisory data include information indicating the synovial region in the ultrasonic image data.

The detection function 383 updates the parameter data PD1 such that a result obtained by processing learning data by a neural network NN approaches the supervisory data whenever the training data set is given, that is, the detection function 383 performs learning. Generally, when a change rate of the parameter data PD1 converges within a threshold, it is determined that the learning is completed. Hereinafter, the parameter data PD1 after the completion of the learning is particularly referred to as learned parameter data PD1t.

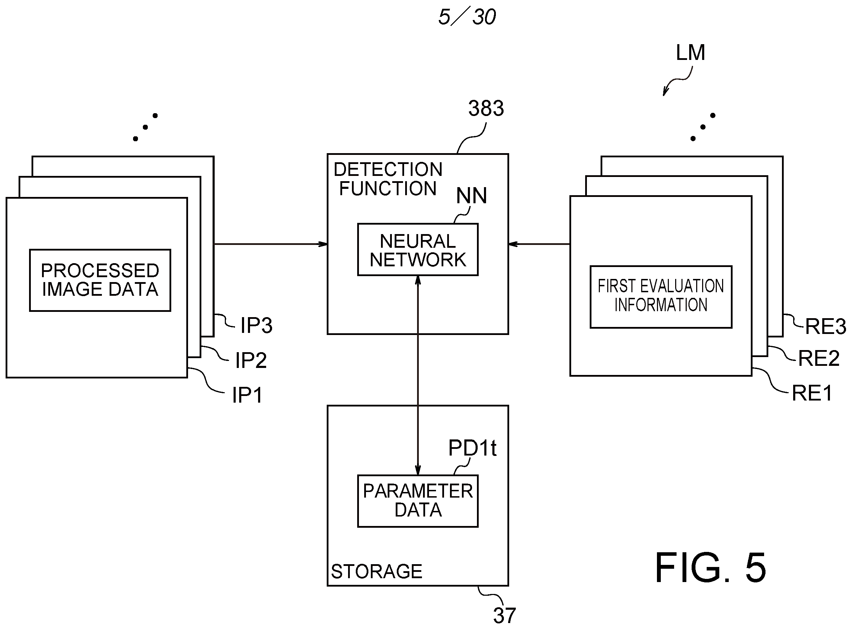

FIG. 5 is an explanatory diagram for describing an example of a data flow at the time of operation (inference) of the detection function 383. The detection function 383 detects a synovial region in each of a plurality of pieces of processed image data different from each other obtained by executing image processing on the ultrasonic image data acquired by the acquisition function 384 by inputting the plurality of pieces of processed image data to the trained model.

The learned parameter data PD1t and the neural network NN constitute a trained model LM. Various methods can be used as this type of learning method and trained model construction method. The neural network NN is stored in the storage 37 in the form of a program. The learned parameter data PD1t may be stored in the storage 37 or may be stored in a storage medium connected to the processing circuitry 38. When the trained model LM (the neural network NN and the learned parameter data PD1t) is stored in the storage 37, the detection function 383 realized by the processor of the processing circuitry 38 can input the processed image data to the trained model LM and detect the synovial region that is the first evaluation information by reading and executing the trained model LM from the storage 37. The trained model LM may be constructed by an integrated circuit such as an application specific integrated circuit (ASIC) or a field programmable gate array (FPGA).

The image processing is processing using at least one of a median filter, a Gaussian filter, an edge enhancement filter, and a non-uniform filter that enhances a high luminance region continuous in one direction of the ultrasonic image data. Alternatively, the image processing is processing using a plurality of preset luminance conversion parameters which are luminance conversion parameters for converting luminance of the ultrasonic image data. Hereinafter, a case where the processed image data is an image obtained by executing processing using the plurality of preset luminance conversion parameters as the image processing on the ultrasonic image data will be described.

The acquisition function 384 acquires ultrasonic image data indicating the joint part (target part) of the subject. The first evaluation information acquisition function 385 acquires the first evaluation information. The first evaluation information is information for evaluating the state of rheumatism in the joint part indicated by the ultrasonic image data. The first evaluation information is, for example, the synovial region in the joint part.

The second evaluation information acquisition function 386 acquires the second evaluation information. The second evaluation information is information for evaluating the state of rheumatism in the joint part. The second evaluation information is, for example, a position where the blood flow signal is present (a position of a pixel indicating the blood flow signal), a straight line connecting the bone surface in the joint part, and the like.

The index value calculation function 387 calculates an index value based on information including at least the first evaluation information and the second evaluation information. Hereinafter, a case where the index value calculation function 387 calculates, as the index value, the ratio of the number of pixels of the blood flow signal in the synovial region to the number of pixels of the synovial region will be described.

The registration function 388 registers the preset information including the imaging order as the protocol. For example, when the setting of the preset information IF1 is completed on the preset information setting screen SC1 illustrated in FIG. 2 and when the user selects the first button B1 via the input device 50, the registration function 388 registers the preset information IF1 as the protocol.

The communication circuitry 39 is connected to, for example, an external apparatus such as a server equipped with picture archiving and communication system (PACS) and a workstation capable of executing post processing via a network, and performs data communication with the external apparatus.

The input device 50 is an input device for performing various settings and the like, and is realized by, for example, a trackball, a switch button, a mouse, a keyboard, a touch pad that performs an input operation by touching an operation surface, a touch monitor in which a display screen and a touch pad are integrated, a non-contact input circuit using an optical sensor, a sound input circuit, and the like. The input device 50 is connected to the processing circuitry 38 of the apparatus main body 30, converts an input operation accepted from the operator into an electric signal, and outputs the electric signal to the processing circuitry 38 of the apparatus main body 30. Note that, in the present specification, the input device 50 is not limited to the device including physical operation components such as a mouse and a keyboard. For example, electric signal processing circuitry that accepts an electric signal corresponding to an input operation from an external input device provided separately from the apparatus and outputs the electric signal to the processing circuitry 38 of the apparatus main body 30 is also included in the example of the input device 50.

The display 70 is a display apparatus that displays various ultrasonic images, various settings, and the like. For example, the display 70 displays ultrasonic images based on the various types of ultrasonic image data generated by the apparatus main body 30, a graphical user interface (GUI) for accepting various operations from the user, and the like. In the present embodiment, the display 70 includes, for example, a liquid crystal display, a cathode ray tube (CRT) display, or the like.

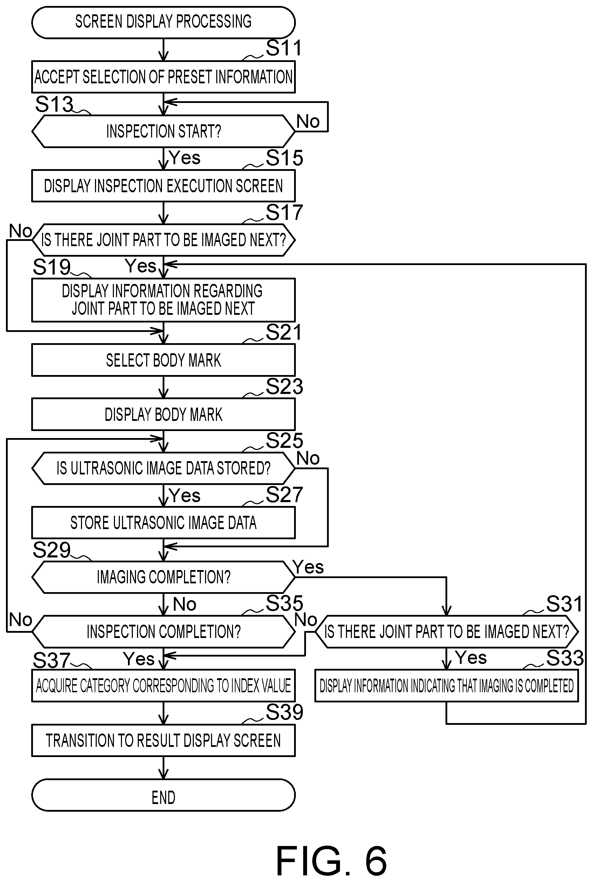

FIG. 6 is a flowchart for describing screen display processing executed in the ultrasonic diagnostic apparatus 1 according to the first embodiment. In this screen display processing, an inspection execution screen is displayed, the information regarding the joint part to be imaged next is displayed, the information indicating that imaging is completed is displayed, and transition to a result display screen is performed. For example, the screen display processing is processing executed when the selection of the preset information is started.

As illustrated in FIG. 6, first, the display control function 382 in the processing circuitry 38 of the apparatus main body 30 accepts the selection of the preset information (step S11). Specifically, the display control function 382 accepts the selection of the preset information by accepting an input operation for selecting the preset information from the user.

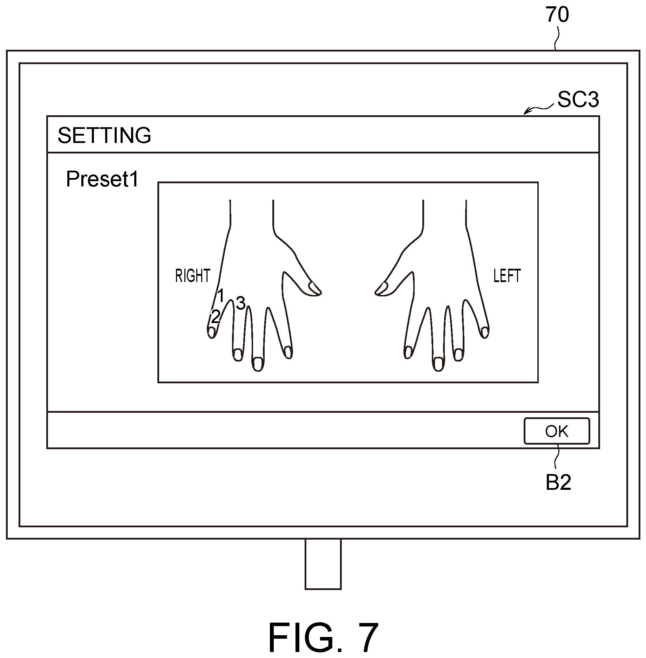

FIG. 7 is a diagram illustrating an example of a preset information selection screen. As illustrated in FIG. 7, the preset information is displayed on a preset information selection screen SC3. A preset for imaging the joint of the little finger of the right hand and the joint of the ring finger of the right hand is displayed in the preset information displayed on the preset information selection screen SC3 illustrated in FIG. 7. In this state, the display control function 382 accepts the selection of the preset information by accepting an operation of pressing a second button B2, as an input operation for selecting the preset information.

Next, as illustrated in FIG. 6, the system control function 381 in the processing circuitry 38 of the apparatus main body 30 determines whether or not inspection is started (step S13). Specifically, the system control function 381 determines whether or not inspection is started by determining whether or not the ultrasonic wave transmission is started.

Next, as illustrated in FIG. 6, the display control function 382 displays the inspection execution screen (step S15). Specifically, the display control function 382 displays the inspection execution screen based on the preset information selected in step S11 on the display 70.

Next, as illustrated in FIG. 6, the display control function 382 determines whether or not there is the joint part to be imaged next (step S17). Specifically, the display control function 382 determines whether or not there is the joint part to be imaged next based on the preset information selected in step S11.

In step S17, when it is determined that there is the joint part to be imaged next (step S17: Yes), the display control function 382 displays the information regarding the joint part to be imaged next, as the information based on the imaging order (step S19). Specifically, the display control function 382 displays the information regarding the joint part to be imaged next based on the preset information selected in step S11 on the inspection execution screen displayed on the display 70.

On the other hand, in step S17, when it is determined that there is no joint part to be imaged next (step S17: No), or after step S19, the display control function 382 selects a body mark (step S21). Specifically, the display control function 382 selects the body mark corresponding to the imaging order based on the preset information selected in step S11. More specifically, the display control function 382 selects the body mark based on the joint part to be imaged in the imaging order included in the preset information selected in step S11, that is, the joint part to be currently imaged.

Next, as illustrated in FIG. 6, the display control function 382 displays the body mark (step S23). Specifically, the display control function 382 displays the body mark selected in step S21 on the inspection execution screen displayed on the display 70.

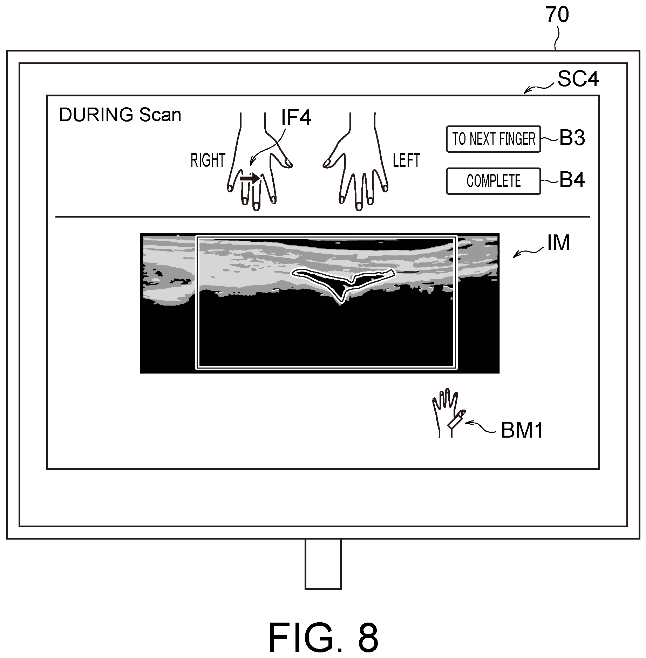

FIG. 8 is a diagram illustrating an example of the inspection execution screen. As illustrated in FIG. 8, information schematically indicating both hands of a human, a third button B3 for transitioning to imaging of a next finger, a fourth button B4 for completing the inspection, information IF4 regarding the joint part to be imaged next, a body mark BM1, and ultrasonic image data IM are displayed on an inspection execution screen SC4. In the example illustrated in FIG. 8, the information IF4 regarding the joint part to be imaged next is an arrow indicating the joint part to be imaged next. In the example illustrated in FIG. 8, "To next finger" is displayed on the third button B3. However, when the joint part to be imaged next is a joint part other than the finger, the content displayed on the third button B3 may be changed according to the joint part to be imaged next, and "To next joint part" may be displayed.

Next, as illustrated in FIG. 6, the display control function 382 determines whether or not the ultrasonic image data is stored (step S25). Specifically, the display control function 382 determines whether or not the ultrasonic image data is stored by determining whether or not an input operation (for example, freeze operation) for storing the ultrasonic image data is accepted from the user via the input device 50.

In step S25, when it is determined that the ultrasonic image data is stored (step S25: Yes), the display control function 382 stores the ultrasonic image data (step S27). Specifically, the display control function 382 stores, in the image memory 36 or the storage 37, the ultrasonic image data displayed on the display 70 when the input operation for storing the ultrasonic image data is accepted from the user in step S25. At this time, the display control function 382 stores the ultrasonic image data by appending the information regarding the joint part based on the imaging order to DICOM data of the ultrasonic image data stored in a DICOM format. The DICOM data is, for example, tag data regarding imaging parameters attached to medical image data in the DICOM format. The information regarding the joint part is information for identifying the joint part, and is, for example, information such as a joint part name and a number assigned to the joint part in the preset information.

On the other hand, in step S25, when it is determined that the ultrasonic image data is not stored (step S25: No), or after step S27, the display control function 382 determines whether or not imaging is completed (step S29). Specifically, the display control function 382 determines whether or not imaging is completed by determining whether or not the user selects the third button B3 for transitioning to the imaging of the next finger, which is displayed on the inspection execution screen SC4, via the input device 50.

In step S29, when it is determined that imaging is completed (step S29: Yes), the display control function 382 determines whether or not there is the joint part to be imaged next, as the information based on the imaging order (step S31). Specifically, the display control function 382 determines whether or not there is the joint part to be imaged next based on the preset information.

In step S31, when there is the joint part to be imaged next (step S31: Yes), the display control function 382 displays the information indicating that imaging is completed (step S33). After the processing of step S33, the processing returns to step S19, and the processing from step S19 is repeated. That is, until inspection is completed or there is no joint part to be imaged next, the processing of displaying the information regarding the joint part to be imaged next (step S19), the processing of selecting the body mark (step S21), the processing of displaying the body mark (step S23), the processing of determining whether or not the ultrasonic image data is stored (step S25), the processing of storing the ultrasonic image data (step S27), and the processing of determining whether or not the imaging is completed (step S29) are repeated.

On the other hand, in step S29, when it is determined that imaging is not completed (step S29: No), the display control function 382 determines whether or not inspection is completed (step S35). Specifically, the display control function 382 determines whether inspection is completed by determining whether or not the user selects the fourth button B4 for completing inspection, which is displayed on the inspection execution screen SC4, via the input device 50. When it is determined that inspection is not completed (step S35: No), the processing returns to step S25, and the processing from step S25 is repeated.

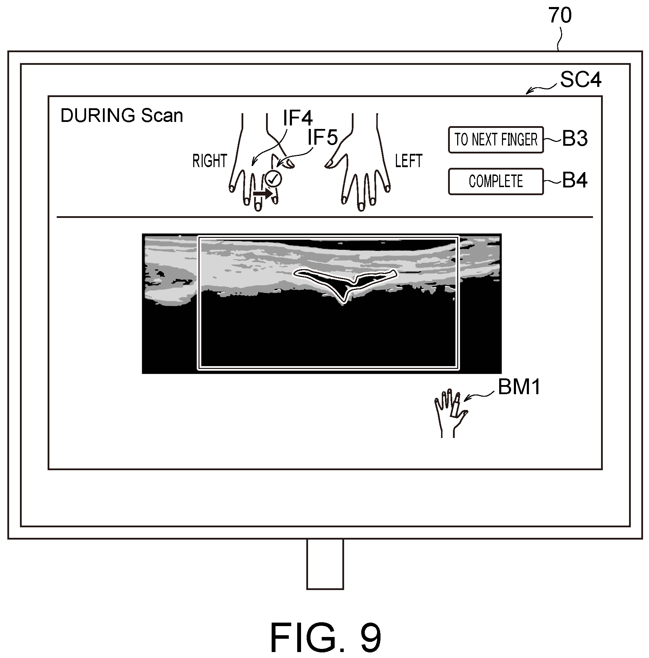

FIG. 9 is a diagram illustrating an example of the inspection execution screen including the information indicating that imaging is completed. As illustrated in FIG. 9, in the information schematically indicating both the hands of the human on the inspection execution screen SC4, a check mark is superimposed and displayed on the joint part where imaging is completed, as information IF5 indicating that imaging is completed. As illustrated in FIG. 9, the display control function 382 changes a display position of the information regarding the joint part to be imaged next within the information schematically indicating both the hands of the human from FIG. 8. As illustrated in FIG. 9, the display control function 382 changes a position of the body mark BM1 to a position of the joint part being imaged.

On the other hand, in step S35, when it is determined that inspection is completed (step S35: Yes), or in step S31, when it is determined that there is no joint part to be imaged next (step S31: No), the display control function 382 acquires the category corresponding to the index value (step S37). Specifically, the display control function 382 acquires the category corresponding to the index value calculated in step S67 of index value calculation processing to be described later from the image memory 36 or the storage 37.

Next, as illustrated in FIG. 6, the display control function 382 transitions to the result display screen (step S39). Specifically, the display control function 382 transitions from the inspection execution screen SC4 to the result display screen.

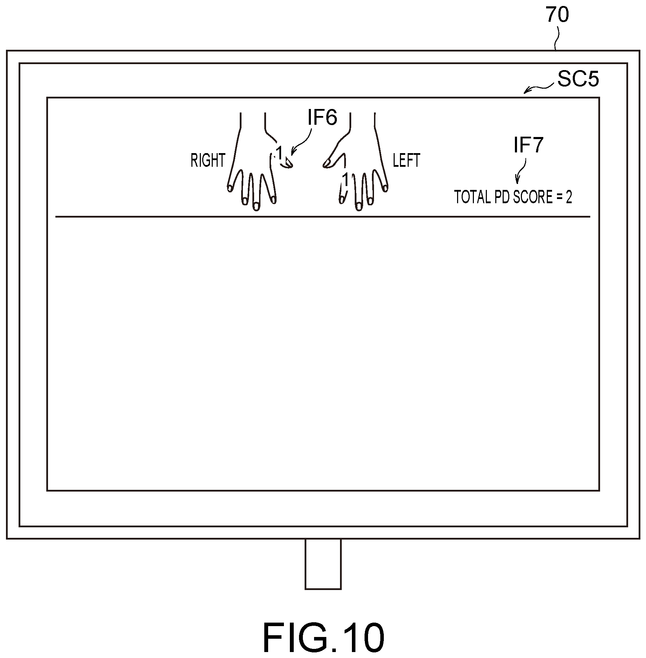

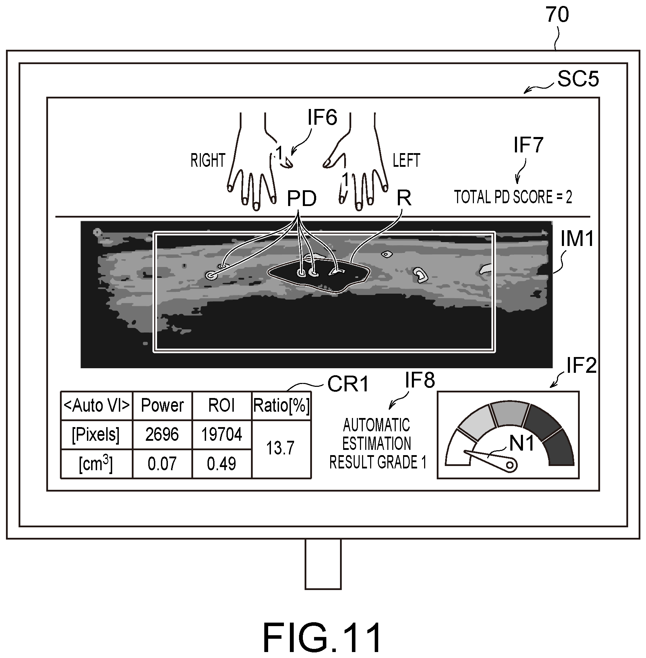

FIG. 10 is a diagram illustrating an example of the result display screen according to the first embodiment, and FIG. 11 is a diagram illustrating an example of the result display screen when a predetermined operation is performed on the result display screen illustrated in FIG. 10. As illustrated in FIG. 10, the information schematically indicating both the hands of the human, information IF6 regarding categories of each of the plurality of joint parts, and information IF7 regarding a sum of the categories of each of the plurality of joint parts are displayed on a result display screen SC5. In the example illustrated in FIG. 10, the information IF6 regarding the categories of each of the plurality of joint parts is a numerical value of the grade. In the example illustrated in FIG. 10, "Grade 1" is displayed on the first joint of the right thumb and "Grade 1" is displayed on the second joint of the left index finger as the information IF6 regarding the categories for each of the plurality of joint parts.

In the example illustrated in FIG. 10, when the inspector performs an input operation of selecting any of the plurality of joint parts in both the hands, as the predetermined operation performed on the result display screen SC5 via the input device 50, the display control function 382 displays, on the result display screen SC5, the ultrasonic image data indicating the joint part, the information capable of identifying the category corresponding to the index value, and the like, which are registered in the selected joint part, as illustrated in FIG. 11. In the example illustrated in FIG. 11, the display control function 382 displays, on the result display screen SC5 on the display 70, ultrasonic image data IM1 indicating the joint part, a synovial region R, a calculation result CR1 of the index value, the information IF2 capable of identifying the category corresponding to the index value, and information IF8 indicating the category corresponding to the index value. Details of the ultrasonic image data IM1 indicating the joint part, the synovial region R, the calculation result CR1 of the index value, the information IF2 capable of identifying the category corresponding to the index value, and the information IF8 indicating the category corresponding to the index value will be described later.

In step S39, the screen display processing is completed by transitioning to the result display screen.

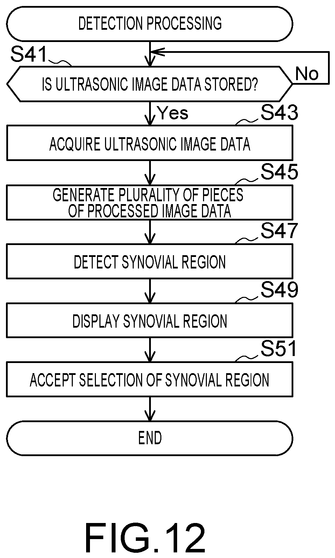

FIG. 12 is a flowchart for describing detection processing executed in the ultrasonic diagnostic apparatus 1 according to the first embodiment. In this detection processing, the plurality of pieces of processed image data is generated, the synovial region is detected, the synovial region is displayed, and the selection of the synovial region is accepted. For example, the detection processing is processing executed when the ultrasonic image data is stored.

As illustrated in FIG. 12, first, the detection function 383 in the processing circuitry 38 of the apparatus main body 30 determines whether or not the ultrasonic image data is stored (step S41). Specifically, the detection function 383 determines whether or not the ultrasonic image data is stored by determining whether or not an input operation for saving the ultrasonic image data is accepted from the user via the input device 50. Then, in step S41, when it is determined that the ultrasonic image data is not stored (step S41: No), the detection function 383 repeats the processing of step S41 and waits until the ultrasonic image data is stored.

On the other hand, in step S41, when it is determined that the ultrasonic image data is saved (step S41: Yes), the detection function 383 acquires the ultrasonic image data (step S43). Specifically, the detection function 383 acquires the ultrasonic image data from the image memory 36 or the storage 37.

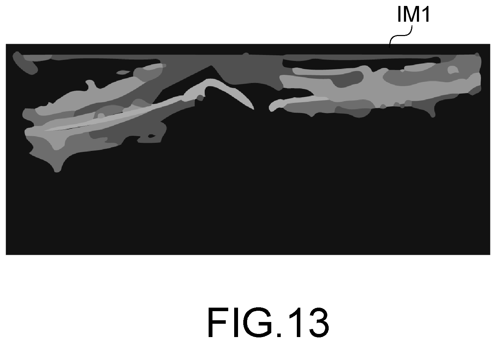

FIG. 13 is a diagram illustrating an example of the ultrasonic image data according to the first embodiment. In the example illustrated in FIG. 11, the detection function 383 acquires the ultrasonic image data IM1 indicating the finger joint as the joint part.

Next, as illustrated in FIG. 12, the detection function 383 generates the plurality of pieces of processed image data (step S45). Specifically, the detection function 383 generates the plurality of pieces of processed image data different from each other by executing image processing on the ultrasonic image data acquired in step S43. More specifically, the detection function 383 generates a plurality of pieces of processed image data having luminances different from each other by executing processing using a plurality of preset luminance conversion parameters on the ultrasonic image data.

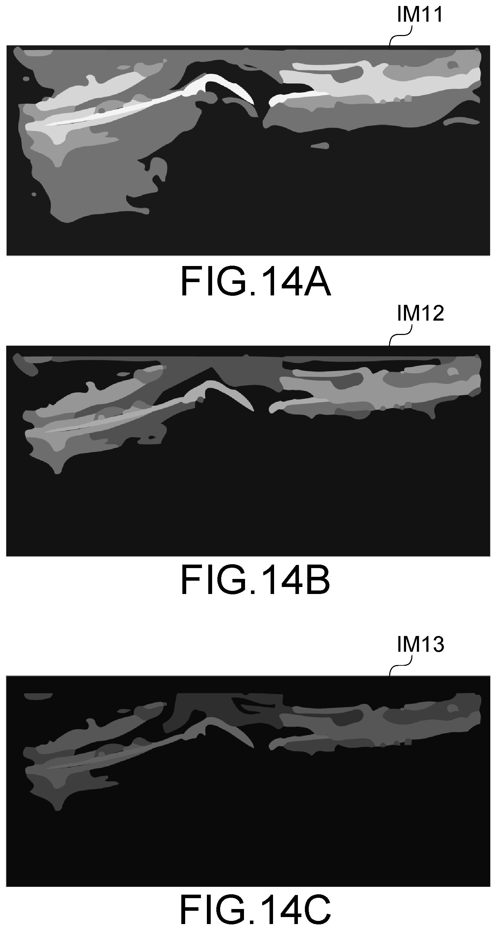

FIGS. 14A to 14C are diagrams illustrating an example of the plurality of pieces of processed image data according to the first embodiment. As illustrated in FIG. 14A, first processed image data IM11 is ultrasonic image data on which image processing is performed by using the luminance conversion parameter so as to achieve high luminance. As illustrated in FIG. 14B, second processed image data IM12 is ultrasonic image data on which image processing is performed by using the luminance conversion parameter so as to have medium luminance. As illustrated in FIG. 14C, third processed image data IM13 is ultrasonic image data on which image processing is performed by using the luminance conversion parameter so as to have low luminance. In the example illustrated in FIG. 14, the detection function 383 generates three pieces of processed image data from the ultrasonic image data, but the number of pieces of processed image data generated from the ultrasonic image data is not limited to three. That is, the number of pieces of processed image data is arbitrary, and may be two or four or more.

Next, as illustrated in FIG. 12, the detection function 383 detects the synovial region (step S47). Specifically, the detection function 383 detects the synovial region in each of the plurality of pieces of processed image data generated in step S45. More specifically, the detection function 383 detects the synovial region in each of the plurality of pieces of processed image data by inputting each of the plurality of pieces of processed image data to the trained model LM.

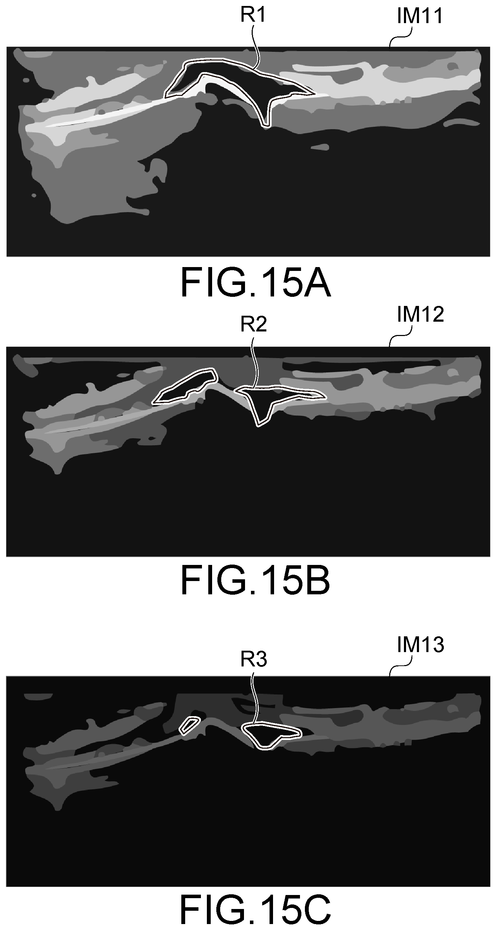

FIGS. 15A to 15C are diagrams illustrating an example of the synovial region detected in each of the plurality of pieces of processed image data according to the first embodiment. A first synovial region R1 illustrated in FIG. 15A is a synovial region detected by inputting the first processed image data IM11 to the trained model LM. A second synovial region R2 illustrated in FIG. 15B is a synovial region detected by inputting the second processed image data IM12 to the trained model LM. A third synovial region R3 illustrated in FIG. 15C is a synovial region detected by inputting the third processed image data IM13 to the trained model LM.

Next, as illustrated in FIG. 12, the display control function 382 displays the synovial region (step S49). Specifically, the display control function 382 displays, on the display 70, the synovial region corresponding to each of the plurality of pieces of processed image data detected in step S47. More specifically, the display control function 382 generates a plurality of pieces of candidate image data by superimposing each of the synovial regions corresponding to each of the plurality of pieces of processed image data detected by the detection function 383 on the ultrasonic image data IM1, and displays the synovial regions by displaying the plurality of pieces of candidate image data.

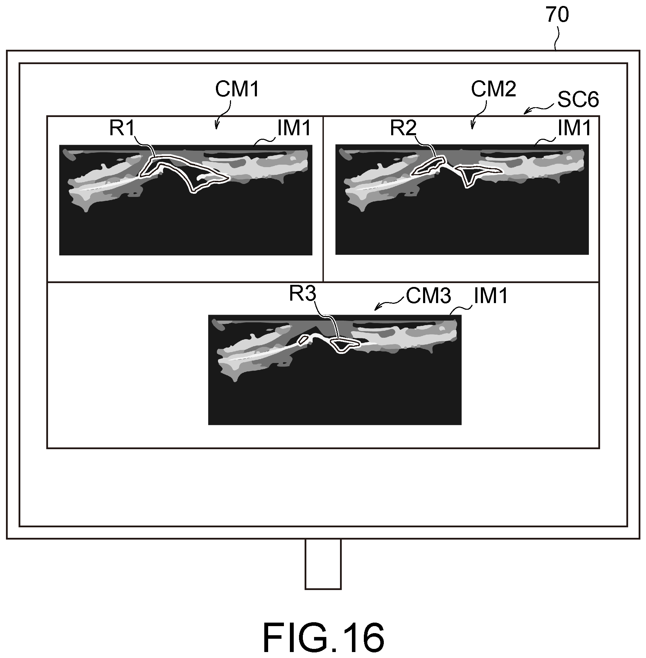

FIG. 16 is a diagram illustrating an example of a detection result display screen. As illustrated in FIG. 16, the display control function 382 displays, on a detection result display screen SC6, first candidate image data CM1 in which the first synovial region R1 is superimposed on the ultrasonic image data IM1, second candidate image data CM2 in which the second synovial region R2 is superimposed on the ultrasonic image data IM1, and third candidate image data CM3 in which the third synovial region R3 is superimposed on the ultrasonic image data IM1 side by side. As described above, the display control function 382 displays the plurality of pieces of candidate image data on one screen, and thus, the user can compare the plurality of pieces of candidate image data.

In the example illustrated in FIG. 16, the display control function 382 displays the plurality of pieces of candidate image data side by side on the detection result display screen SC6, but a method for displaying the plurality of pieces of candidate image data is not limited thereto. That is, the method for displaying the plurality of pieces of candidate image data is arbitrary, and for example, the display control function 382 may display one candidate image data of the plurality of pieces of candidate image data on the detection result display screen SC6, and switch the candidate image data to be displayed on the detection result display screen SC6 among the plurality of pieces of candidate image data by accepting an input operation of switching the one candidate image data to be displayed on the detection result display screen SC6 via the input device 50 from the user.

In the example illustrated in FIG. 16, the plurality of pieces of candidate image data is generated by superimposing each of the plurality of synovial regions detected by the detection function 383 on the ultrasonic image data IM1, but a method for generating the plurality of pieces of candidate image data is not limited thereto. That is, the method for generating the plurality of pieces of candidate image data is arbitrary, and for example, the display control function 382 may generate the plurality of pieces of candidate image data by superimposing each of the plurality of synovial regions detected by the detection function 383 on the processed image data corresponding to each of the plurality of synovial regions.

Next, as illustrated in FIG. 12, the display control function 382 accepts the selection of the synovial region (step S51). Specifically, the display control function 382 accepts the selection of the synovial region by accepting an input operation of accepting the selection of the synovial region via the input device 50 from the user. More specifically, the display control function 382 accepts the selection of the synovial region by accepting an input operation of accepting the selection of the plurality of pieces of candidate image data displayed on the display 70 from the user. The candidate image data corresponding to the selected synovial region is stored in the image memory 36 or the storage 37.

In step S51, the selection of the synovial region is accepted, and thus, synovial region detection processing is completed.

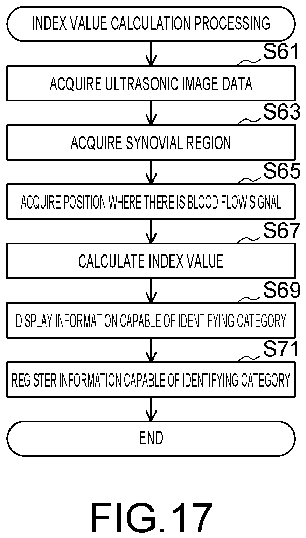

FIG. 17 is a flowchart for describing index value calculation processing executed in the ultrasonic diagnostic apparatus 1 according to the first embodiment. In this index value calculation processing, the ultrasonic image data is acquired, the synovial region is acquired, the position where the blood flow signal is present is acquired, the index value is calculated, the information capable of identifying the category is displayed, and the information capable of identifying the category is registered. For example, the index value calculation processing is processing executed when inspection is completed. The index value calculation processing may be processing executed when the ultrasonic image data is stored, or may be processing executed when the selection of the synovial region is accepted.

As illustrated in FIG. 17, first, the acquisition function 384 in the processing circuitry 38 of the apparatus main body 30 acquires the ultrasonic image data (step S61). Specifically, the acquisition function 384 acquires the ultrasonic image data indicating the joint part of the subject from the image memory 36 or the storage 37. More specifically, the acquisition function 384 acquires, as the ultrasonic image data indicating the joint part of the subject, an image in which the B mode image data indicating the joint part of the subject and the blood flow image (power Doppler image) PD that is an image indicating the blood flow signal are combined.

Next, as illustrated in FIG. 17, the first evaluation information acquisition function 385 in the processing circuitry 38 of the apparatus main body 30 acquires the synovial region (step S63). Specifically, the first evaluation information acquisition function 385 acquires the synovial region as the first evaluation information by acquiring the candidate image data selected in step S51 of the synovial region detection processing.

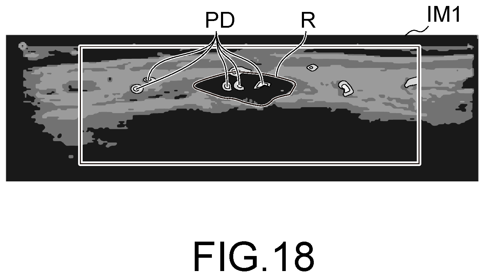

FIG. 18 is a diagram illustrating an example of the ultrasonic image data and the synovial region. As illustrated in FIG. 18, the ultrasonic image data IM1 is an image obtained by combining the B mode image data and the blood flow image (power Doppler image) PD that is the image indicating the blood flow signal, and the first synovial region R1 is superimposed on the ultrasonic image data IM1.

Next, as illustrated in FIG. 17, the second evaluation information acquisition function 386 in the processing circuitry 38 of the apparatus main body 30 acquires the position where the blood flow signal is present (step S65). Specifically, the second evaluation information acquisition function 386 acquires, as the second evaluation information, a position of the ultrasonic image data where the blood flow signal is present from the ultrasonic image data acquired in step S61. In step S65, the second evaluation information acquisition function 386 acquires, as the second evaluation information, the position of the ultrasonic image data where the blood flow signal is present, but a target to be acquired by the second evaluation information acquisition function 386 is not limited to the position where the blood flow signal is present. That is, the target to be acquired by the second evaluation information acquisition function 386 is arbitrary, and the number of blood flow pixels in the synovial region may be acquired.

Next, as illustrated in FIG. 17, the index value calculation function 387 in the processing circuitry 38 of the apparatus main body 30 calculates the index value (step S67). Specifically, the index value calculation function 387 calculates the index value indicating the state of rheumatism based on information including at least the synovial region and the position where the blood flow signal is present. More specifically, the index value calculation function 387 calculates, as the index value, the ratio of the number of pixels of the blood flow signal in the synovial region to the number of pixels of the synovial region based on the number of pixels of the synovial region and the number of pixels of the blood flow signal in the synovial region.

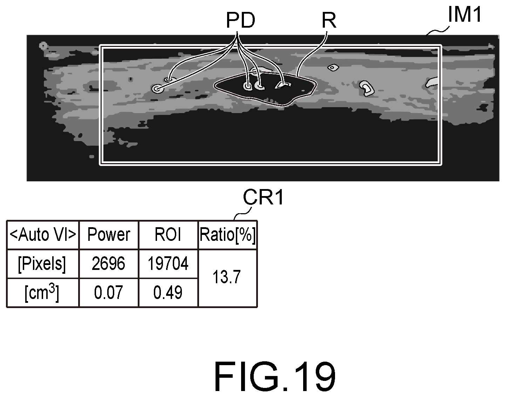

FIG. 19 is a diagram illustrating an example of the calculation result of the index value in the ultrasonic image data illustrated in FIG. 18. As illustrated in FIG. 19, the calculation result CR1 of the index value in the ultrasonic image data illustrated in FIG. 18 indicates that the number of pixels of the blood flow signal in the first synovial region R1 is "2696", the number of pixels of the first synovial region R1 is "19704", and the ratio of the number of pixels of the blood flow signal in the synovial region to the number of pixels of the synovial region is "13.7". That is, the index value calculation function 387 calculates, as the index value, a ratio "13.7" of the number of pixels of the blood flow signal in the synovial region to the number of pixels in the synovial region.

Next, as illustrated in FIG. 17, the display control function 382 displays the information capable of identifying the category (step S69). Specifically, the display control function 382 displays an indicator capable of identifying the category corresponding to the index value, as the information IF2 capable of identifying the category corresponding to the index value calculated in step S67.

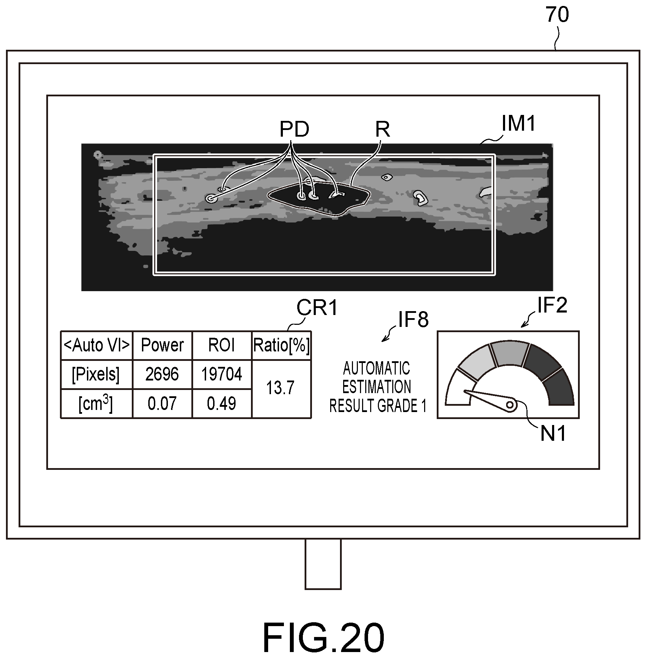

FIG. 20 is a diagram illustrating an example of the information capable of identifying the category corresponding to the index value illustrated in FIG. 19. As illustrated in FIG. 20, the display control function 382 displays, on the display 70, ultrasonic image data IM2, the synovial region R, the plurality of graduations corresponding to the plurality of categories, the indicator including the indicator needle N1, and the information IF8 indicating the category corresponding to the index value together with the calculation result of the index value. The configuration of each of the plurality of gradations is equivalent to the indicator illustrated in FIG. 3, and thus, the description thereof will be omitted. In the example illustrated in FIG. 20, since the index value is calculated to be "13.7" and this index value is the second category indicating grade 1 in the threshold information IF3 illustrated in FIG. 3, the indicator needle N1 indicates the first graduation CA1 indicating the second category.

Next, as illustrated in FIG. 17, the display control function 382 registers the information IF2 capable of identifying the category (step S71). Specifically, the display control function 382 registers the information IF2 capable of identifying the category corresponding to the index value, for the joint part corresponding to the ultrasonic image data. In the present embodiment, the display control function 382 registers the ultrasonic image data IM2, the synovial region R, the calculation result of the index value, and the information IF8 indicating the category corresponding to the index value, for the joint part corresponding to the ultrasonic image data, together with the information IF2 capable of identifying the category corresponding to the index value.

In step S71, the index value calculation processing is completed by registering the information IF2 capable of identifying the category.

As described above, the ultrasonic diagnostic apparatus 1 according to the first embodiment acquires the ultrasonic image data indicating the joint part of the subject, acquires the synovial region, acquires the position where the blood flow signal is present, calculates the index value based on the synovial region and the position where the blood flow signal is present, and displays the information capable of identifying the category corresponding to the index value. Therefore, it is possible to evaluate the state of rheumatism with a fine level of granularity and to reduce a degree of dependence on the inspector in the diagnosis of rheumatism.

### [Modification 1]

The detection processing executed in the ultrasonic diagnostic apparatus 1 according to the first embodiment described above can be executed when the acquisition function 384 acquires medical image data other than the ultrasonic image data, that is, when a medical image diagnostic apparatus other than the ultrasonic image diagnostic apparatus is used, and when the detection function 383 detects the first evaluation information other than the synovial region. In this case, the medical image diagnostic apparatus acquires the medical image data in step S41 of the detection processing, generates the plurality of pieces of processed image data in step S43 of the detection processing, detects the first evaluation information in step S45 of the detection processing, displays the first evaluation information in step S47 of the detection processing, and accepts the selection of the first evaluation information.

The medical image diagnostic apparatus is arbitrary, and may be, for example, an X-ray diagnostic apparatus such as, for example, a general X-ray diagnostic apparatus or an X-ray computed tomography (CT) apparatus, a magnetic resonance imaging (MRI) apparatus, a single photon emission computed tomography (SPECT) apparatus, or a positron emission computed tomography (PET) apparatus. That is, processing similar to the detection processing executed by the processing circuitry 38 of the apparatus main body 30 may be executed on medical image data such as X-ray image data, MRI image data, SPECT image data, and PET image data acquired in the medical image diagnostic apparatus.

### [Modification 2]

On the result display screen SC5 in the ultrasonic diagnostic apparatus 1 according to the first embodiment described above, the numerical value of the grade is displayed as the information IF6 regarding the categories of each of the plurality of joint parts so as to be superimposed on the information schematically indicating both the hands of the human. However, the information IF6 regarding the categories of each of the plurality of joint parts displayed on the result display screen SC5 can also display the visual information assigned to the category corresponding to the index value so as to be superimposed.

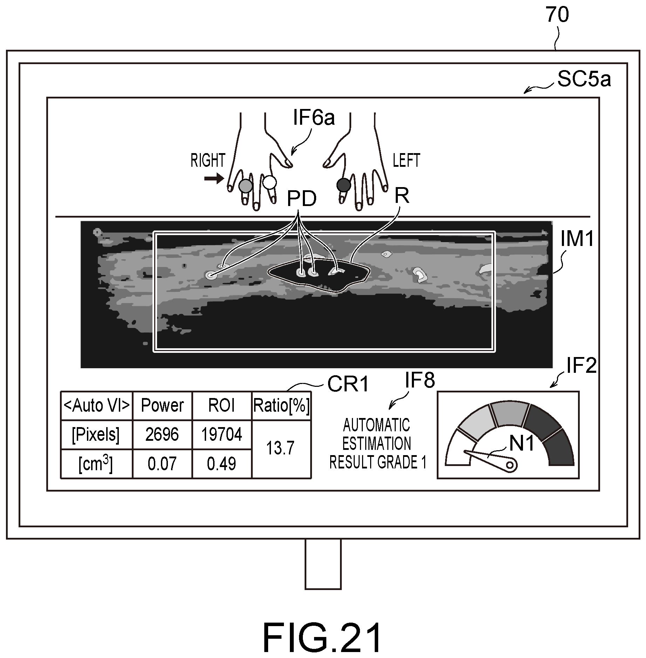

FIG. 21 is a diagram illustrating an example of a result display screen according to Modification 2. As illustrated in FIG. 21, information schematically indicating both hands of a human, information IF6a regarding categories of each of a plurality of joint parts, ultrasonic image data IM1 indicating the joint part, a synovial region R, a calculation result CR1 of an index value, information IF2 capable of identifying a category corresponding to the index value, and information IF8 indicating the category corresponding to the index value are displayed on a result display screen SC5a. Information IF7 regarding a sum of the categories of each of the plurality of joint parts may be displayed on the result display screen SC5a.

In the example illustrated in FIG. 21, as the information IF6a regarding the categories of each of the plurality of joint parts, a green circle assigned to the second category corresponding to grade 1 is displayed in a first joint of an index finger of the right hand, a yellow circle assigned to the fourth category corresponding to grade 2 is displayed in a first joint of a ring finger of the right hand, and a red circle assigned to the sixth category corresponding to grade 3 is displayed in a first joint of an index finger of the left hand. As described above, even when the visual information assigned to the category corresponding to the index value is superimposed and displayed, the inspector can identify the state of rheumatism with a fine level of granularity.

### [Modification 3]

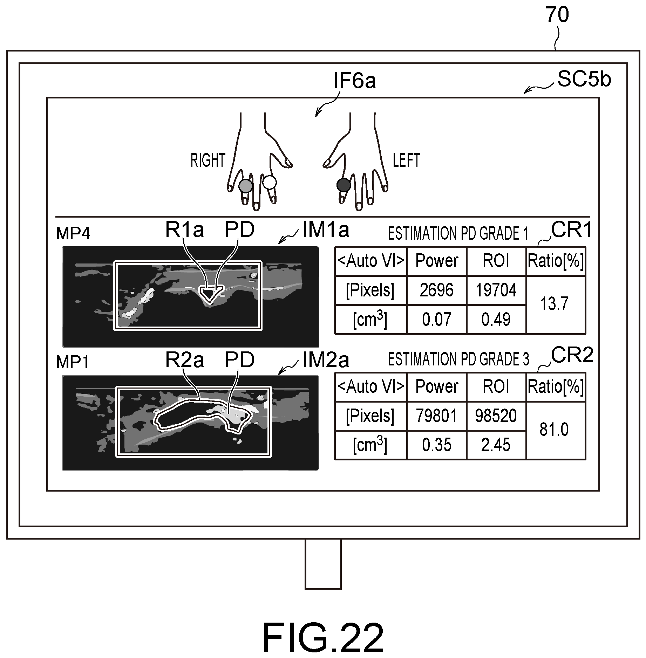

On the result display screen SC5 in the ultrasonic diagnostic apparatus 1 according to the first embodiment described above, it is also possible to display the ultrasonic image data of each of two different joint parts in the same inspection and the calculation result of the index value. FIG. 22 is a diagram illustrating an example of a result display screen according to Modification 3. As illustrated in FIG. 22, information schematically indicating both hands of a human, information IF6a regarding categories of each of a plurality of joint parts, first ultrasonic image data IM1a, a first synovial region R1a, a calculation result CR1 of an index value in the first ultrasonic image data IM1a, second ultrasonic image data IM2a, a second synovial region R2a, and a calculation result CR2 of an index value in the second ultrasonic image data IM2a are displayed on a result display screen SC5b. The first ultrasonic image data IM1a, the first synovial region R1a, the calculation result CR1 of the index value in the first ultrasonic image data IM1a, the second ultrasonic image data IM2a, the second synovial region R2a, and the calculation result CR2 of the index value in the second ultrasonic image data IM2a are displayed, and thus, the inspector can compare and evaluate the inspection results of the plurality of joint parts.

### [Modification 4]

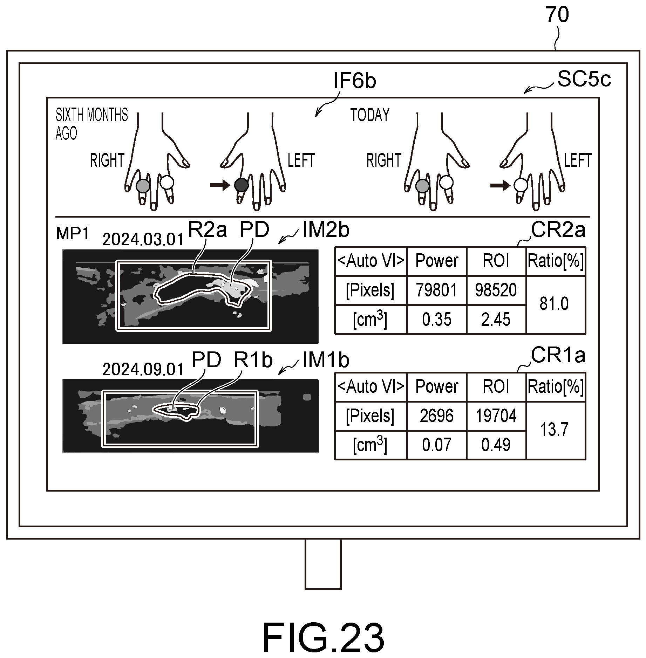

On the result display screen SC5 in the ultrasonic diagnostic apparatus 1 according to the first embodiment described above, it is also possible to display the past inspection result and the current inspection result in a comparable manner. FIG. 23 is a diagram illustrating an example of a result display screen according to Modification 4. As illustrated in FIG. 23, information schematically indicating both hands of a human, information IF6b regarding categories of each of a plurality of joint parts, first ultrasonic image data IM1b, a first synovial region R1b, a calculation result CR1a of an index value in the first ultrasonic image data IM1b, second ultrasonic image data IM2b, a second synovial region R2a, and a calculation result CR2a of an index value in the second ultrasonic image data IM2b are displayed on a result display screen SC5c. The display control function 382 displays the first ultrasonic image data IM1b, the first synovial region R1b, the calculation result CR1a of the index value in the first ultrasonic image data IM1b, the second ultrasonic image data IM2b, the second synovial region R2a, and the calculation result CR2a of the index value in the second ultrasonic image data IM2b side by side by acquiring the past inspection result regarding the same joint part as the current inspection result from the image memory 36, the storage 37, or an external storage device based on the information regarding the joint part based on the imaging order attached to the DICOM data. As described above, the current inspection result and the past inspection result are displayed side by side, and thus, the inspector can compare and evaluate the inspection result of the current joint part with the inspection result of the past joint part.

### [Modification 5]

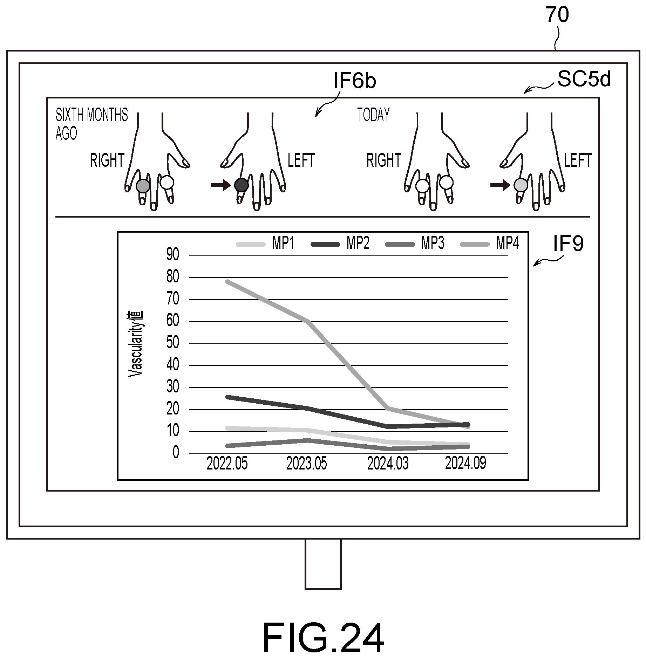

The result display screen SC5 in the ultrasonic diagnostic apparatus 1 according to the first embodiment described above can also display information regarding time-series data of the index value. FIG. 24 is a diagram illustrating an example of a result display screen according to Modification 5. As illustrated in FIG. 24, information schematically indicating both hands of a human, information IF6b regarding categories of each of a plurality of joint parts, and information IF9 regarding time-series data of an index value are displayed on a result display screen SC5d. In the example illustrated in FIG. 24, the information IF9 regarding the time-series data of the index value is a line graph in which a vertical axis is a Vascularity value and a horizontal axis is an inspection year and month. The display control function 382 generates this line graph for each joint part by acquiring the past inspection result regarding the same joint part as the current inspection result from the image memory 36, the storage 37, or an external storage device based on the information regarding the joint part based on the imaging order attached to the DICOM data. As described above, the current inspection result, the past inspection result, and the line graph are displayed, and thus, the inspector can evaluate the inspection result of the current joint part and the inspection result of the past joint part in time series.

In the line graph in the ultrasonic diagnostic apparatus 1 according to Modification 5 described above, the vertical axis represents the Vascularity value, but the present disclosure is not limited thereto. The vertical axis of the line graph may be a grade value, and the grade value and the Vascularity value may be switched.

### [Modification 6]

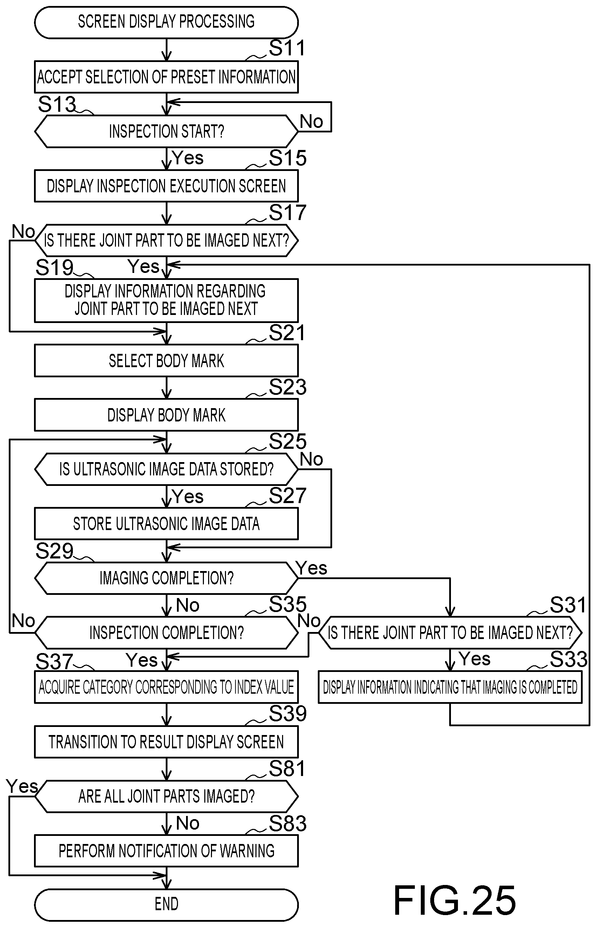

In the ultrasonic diagnostic apparatus 1 according to the first embodiment described above, it is also possible to notify a warning when not all joint parts are imaged in the case of transitioning to the result display screen. FIG. 25 is a flowchart for describing screen display processing executed in an ultrasonic diagnostic apparatus 1 according to Modification 6. In this screen display processing, the inspection execution screen is displayed, the information regarding the joint part to be imaged next is displayed, the information indicating that imaging is completed is displayed, the transition to the result display screen is performed, whether or not all the joint parts are imaged is determined, and the warning is notified. For example, the screen display processing is processing executed when the selection of the preset information is started. The processing from step S11 to step S39 of the screen display processing according to Modification 6 is the same as that of the first embodiment described above, and thus, the description thereof will be omitted.

Next, as illustrated in FIG. 25, the display control function 382 determines whether or not all the joint parts are imaged (step S81). Specifically, the display control function 382 determines whether or not all the joint parts for which the imaging order related to the preset information is set are imaged based on the preset information for which selection is accepted in step S11 and the information indicating that imaging is completed or the DICOM data of the ultrasonic image data stored in the storage 37.

In step S81, when all the joint parts are not imaged (step S81: No), the display control function 382 notifies the warning (step S83). Specifically, the display control function 382 notifies the warning by displaying, on the display 70, information indicating that there is a joint part that is not imaged.

On the other hand, when all the joint parts are imaged in step S81 (step S81: Yes), or the warning is notified in step S83, the screen display processing according to Modification 6 is completed.

As described above, in the ultrasonic diagnostic apparatus 1 according to Modification 6, it is determined whether or not all the joint parts are imaged, and the warning is notified when all the joint parts are not imaged. Therefore, it is possible to reduce a possibility of omission of inspection.

### [Modification 7]

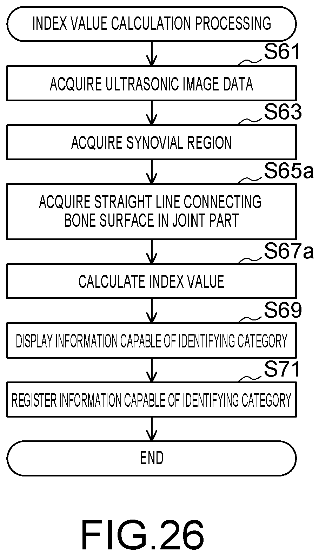

In the ultrasonic diagnostic apparatus 1 according to the first embodiment described above, the case where the second evaluation information is the position where the blood flow signal is present and the index value is the ratio of the number of pixels of the blood flow signal in the synovial region to the number of pixels of the synovial region has been described. However, the second evaluation information may be a straight line connecting the bone surface in the joint part, and the index value may be the ratio of the number of pixels of the synovial region that does not exceed the straight line of the bone surface to the number of pixels of the synovial region that exceeds the straight line of the bone surface. FIG. 26 is a flowchart for describing index value calculation processing executed in an ultrasonic diagnostic apparatus 1 according to Modification 7. In this index value calculation processing, the ultrasonic image data is acquired, the synovial region is acquired, the straight line connecting the bone surface in the joint part is acquired, the index value is calculated, the information capable of identifying the category is displayed, and the information capable of identifying the category is registered. For example, the index value calculation processing is processing executed when inspection is completed. The index value calculation processing may be processing executed when the selection of the synovial region is accepted. In addition, the processing of steps S61 and S63 is the same as that of the first embodiment described above, and thus, the description thereof will be omitted.

Next, as illustrated in FIG. 26, the second evaluation information acquisition function 386 acquires the straight line connecting the bone surface in the joint part (step S65a). Specifically, the second evaluation information acquisition function 386 acquires, as the second evaluation information, the straight line connecting the bone surface in the joint part by performing image analysis on the ultrasonic image data acquired in step S61.

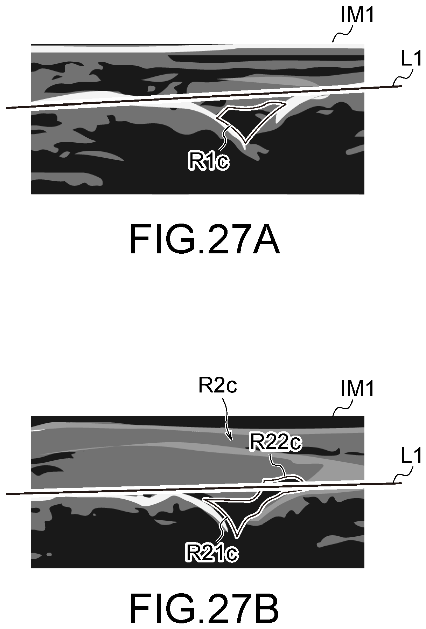

FIGS. 27A and 27B are diagrams illustrating an example of first evaluation information and second evaluation information according to Modification 7, FIG. 27A is a diagram illustrating an example of the synovial region that does not exceed the straight line of the bone surface, and FIG. 27B is a diagram illustrating an example of the synovial region that exceeds the straight line of the bone surface. In B mode image data IM1 which is the ultrasonic image data illustrated in FIG. 27A, a synovial region R1c acquired in step S63 and a straight line L1 connecting the bone surface in the joint part acquired in step S65a are superimposed and displayed. In B mode image data IM1 which is the ultrasonic image data illustrated in FIG. 27B, a synovial region R2c acquired in step S63 and a straight line L1 connecting the bone surface in the joint part acquired in step S65a are superimposed and displayed. In the example illustrated in FIG. 27B, the synovial region R2c includes a portion R21c not exceeding the straight line L1 of the bone surface and a portion R22c exceeding the straight line L1 of the bone surface.

Next, as illustrated in FIG. 26, the index value calculation function 387 calculates the index value (step S67a). Specifically, the index value calculation function 387 calculates, as the index value, the ratio of the number of pixels in the synovial region not exceeding the straight line of the bone surface to the number of pixels in the synovial region exceeding the straight line of the bone surface. More specifically, taking FIG. 27B as an example, the number of pixels of the portion R22c exceeding the straight line L1 of the bone surface to the number of pixels of the portion R21c not exceeding the straight line L1 of the bone surface is calculated. The processing in steps S69 and S71 after step S67a is the same as that in the first embodiment described above, and thus, the description thereof will be omitted.

As described above, in the ultrasonic diagnostic apparatus 1 according to Modification 7, even when the second evaluation information is the straight line connecting the bone surface in the joint part and the index value is the ratio of the number of pixels in the synovial region that does not exceed the straight line of the bone surface to the number of pixels in the synovial region that exceeds the straight line of the bone surface, it is possible to evaluate the state of rheumatism with a fine level of granularity and to reduce the degree of dependence on the inspector in the diagnosis of rheumatism, as in the first embodiment described above.

### [Modification 8]

In the ultrasonic diagnostic apparatus 1 according to the first embodiment described above, it is also possible to give the visual information to at least a part of the synovial region. Specifically, when the synovial region is displayed on the ultrasonic image data on the result display screen SC5, the display control function 382 may give the same visual information as the visual information assigned to the category corresponding to the index value to at least a part of a line surrounding the synovial region. As a result, the inspector can identify the category corresponding to the index value by visually recognizing the synovial region.

### [Second Embodiment]

In the first embodiment and Modification 1 described above, the case where the ultrasonic diagnostic apparatus 1 executes various types of processing has been described, but the medical image processing apparatus may execute processing similar to the processing executed by the ultrasonic diagnostic apparatus 1. Hereinafter, such an embodiment will be referred to as a second embodiment, and differences from the first embodiment will be described. Hereinafter, a case where this modification is applied to the first embodiment will be described, but this modification is also applicable to Modification 1 described above.

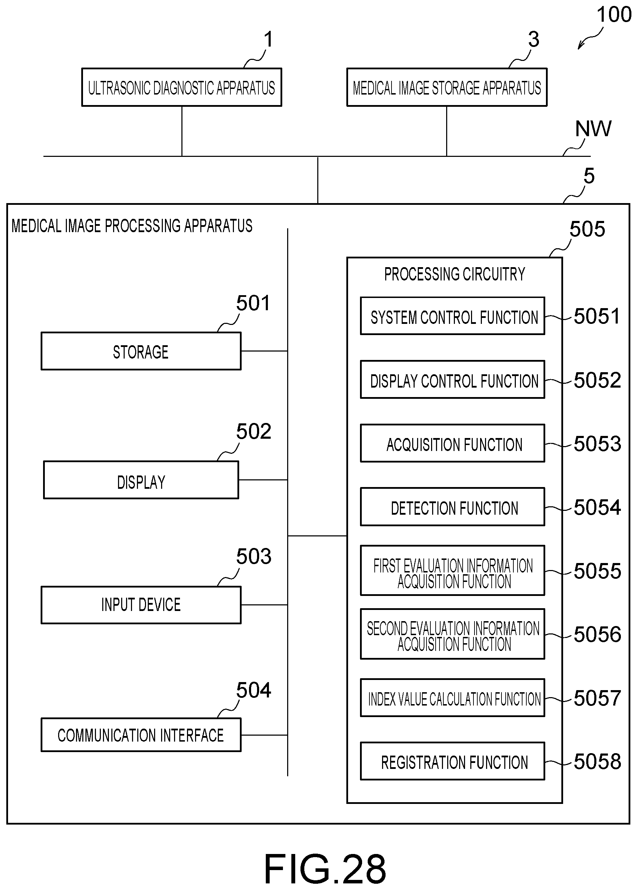

FIG. 28 is a block diagram illustrating a configuration example of a medical image processing system according to the second embodiment. As illustrated in FIG. 28, a medical image processing system 100 includes an ultrasonic diagnostic apparatus 1, a medical image storage apparatus 3, and a medical image processing apparatus 5. As illustrated in FIG. 28, the ultrasonic diagnostic apparatus 1, the medical image storage apparatus 3, and the medical image processing apparatus 5 are connected to communicate via a network NW.

The ultrasonic diagnostic apparatus 1 according to the second embodiment generates ultrasonic image data. The ultrasonic diagnostic apparatus 1 transmits the generated ultrasonic image data to the medical image processing apparatus 5 via the communication circuitry 39.

The medical image storage apparatus 3 stores the ultrasonic image data collected by the ultrasonic diagnostic apparatus 1. For example, the medical image storage apparatus 3 is realized by a computer apparatus such as a server apparatus. The medical image storage apparatus 3 acquires the ultrasonic image data from the ultrasonic diagnostic apparatus 1 via the network NW, and stores the acquired ultrasonic image data in a memory such as a hard disk or an optical disk provided inside or outside the apparatus. The medical image storage apparatus 3 transmits the ultrasonic image data stored in the memory to the medical image processing apparatus 5 in response to a request from the medical image processing apparatus 5.

The medical image processing apparatus 5 acquires the ultrasonic image data from the ultrasonic diagnostic apparatus 1 or the medical image storage apparatus 3 via the network NW, and executes processing similar to the processing executed by the ultrasonic diagnostic apparatus 1 according to the first embodiment on the acquired ultrasonic image data. For example, the medical image processing apparatus 5 executes the screen display processing, the detection processing, and the index value calculation processing. As illustrated in FIG. 28, the medical image processing apparatus 5 includes storage 501, a display 502, an input device 503, communication circuitry 504, and processing circuitry 505.

The storage 501 stores various types of data such as a control program for performing the screen display processing, the detection processing, and the index value calculation processing. The storage 501 is a memory that stores various images acquired from the ultrasonic diagnostic apparatus 1 or the medical image storage apparatus 3. For example, the storage 501 is realized by a semiconductor memory element such as a flash memory, a hard disk, or an optical disk.

The display 502 displays a graphical user interface (GUI) for the user to input various setting requests by using the input device 503, and displays the ultrasonic image data. The display 502 displays various messages in order to notify the user of a processing status.

The input device 503 is connected to the processing circuitry 505, converts an input operation accepted from the user into an electric signal, and outputs the electric signal to the processing circuitry 505. The input device 503 is not limited to a device including physical operation components such as a mouse or a keyboard. For example, processing circuitry that accepts an electric signal corresponding to an input operation from an external input device provided separately from the apparatus and outputs the electric signal to a control circuit is also included in an example of the input device. For example, the input device 503 is realized by a trackball for performing various settings and the like, a switch button, a mouse, a keyboard, a touch pad that performs an input operation by touching on an operating surface, a touch screen in which a display screen and a touch pad are integrated, non-contact input device using an optical sensor, or a sound input device.

The communication circuitry 504 is connected to the processing circuitry 505 and controls transmission of various types of data with the ultrasonic diagnostic apparatus 1 connected via the network NW and communication with the ultrasonic diagnostic apparatus 1. For example, the communication circuitry 504 is realized by a network card, a network adapter, a network interface controller (NIC), or the like.

The processing circuitry 505 executes various types of data processing. The processing circuitry 505 has a system control function 5051, a display control function 5052, a detection function 5053, an acquisition function 5054, a first evaluation information acquisition function 5055, a second evaluation information acquisition function 5056, an index value calculation function 5057, and a registration function 5058. Here, for example, each processing function of the system control function 5051, the display control function 5052, the detection function 5053, the acquisition function 5054, the first evaluation information acquisition function 5055, the second evaluation information acquisition function 5056, the index value calculation function 5057, and the registration function 5058, which are components of the processing circuitry 505 illustrated in FIG. 28, is recorded in the storage 501 in the form of a program executable by a computer. The processing circuitry 505 realizes a function corresponding to each program by reading each program from the storage 501 and executing each read program. In other words, the processing circuitry 505 in a state of reading each program has the function of the processing circuitry 505 illustrated in FIG. 28. The processing circuitry 505 is realized by, for example, a processor.

The system control function 5051 corresponds to the system control function 381 described above. The system control function 5051 performs processing similar to that of the system control function 381. The system control function 5051 is an example of a control unit. The display control function 5052 corresponds to the display control function 382 described above. The display control function 5052 performs processing similar to that of the display control function 382. The display control function 5052 is an example of a display control unit. The detection function 5053 corresponds to the detection function 383 described above. The detection function 5053 performs processing similar to that of the detection function 383. The detection function 5053 is an example of a detection unit. The acquisition function 5054 corresponds to the acquisition function 384 described above. The acquisition function 5054 performs processing similar to that of the acquisition function 384. The acquisition function 5054 is an example of an acquisition unit. The first evaluation information acquisition function 5055 corresponds to the first evaluation information acquisition function 385 described above. The first evaluation information acquisition function 5055 performs processing similar to that of the first evaluation information acquisition function 385. The first evaluation information acquisition function 5055 is an example of a first evaluation information acquisition unit. The second evaluation information acquisition function 5056 corresponds to the second evaluation information acquisition function 386 described above. The second evaluation information acquisition function 5056 performs processing similar to that of the second evaluation information acquisition function 386. The second evaluation information acquisition function 5056 is an example of a second evaluation information acquisition unit. The index value calculation function 5057 corresponds to the index value calculation function 387 described above. The index value calculation function 5057 performs processing similar to that of the index value calculation function 387. The index value calculation function 5057 is an example of an index value calculation unit. The registration function 5058 corresponds to the registration function 388 described above. The registration function 5058 performs processing similar to that of the registration function 388. The registration function 5058 is an example of a registration unit.

As described above, in the medical image processing apparatus 5 in the medical image processing system 100 according to the second embodiment, it is also possible to reduce the degree of dependence on the inspector in the diagnosis of rheumatism, similarly to the ultrasonic diagnostic apparatus 1 described above.

The medical image processing apparatus 5 according to the second embodiment described above may be realized by a workstation or cloud computing. At this time, the display 502 and the input device 503 may be connected to a network as a client device, for example. The storage 501, the communication circuitry 504, and the processing circuitry 505 may be mounted on a server on a network.

### [Other modifications]

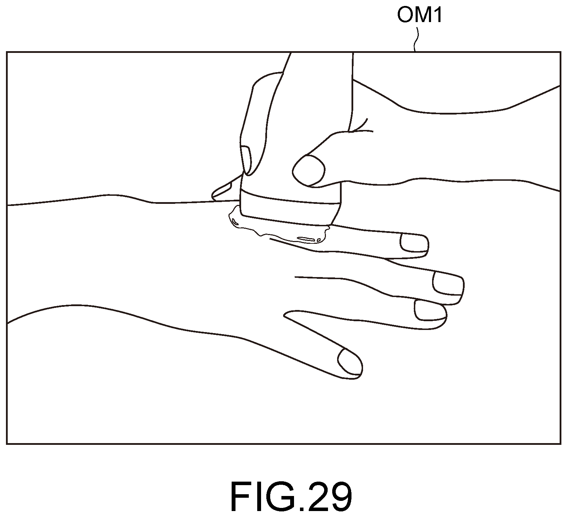

In the ultrasonic diagnostic apparatus 1 according to the first and second embodiments and Modifications 1 to 7 described above, the imaged joint part may be specified by analyzing an optical image imaged by using an optical camera. FIG. 29 is a diagram illustrating an example of the optical image. In the example illustrated in FIG. 29, on an optical image OM1, a second joint of the index finger of the right hand is inspected. The processing circuitry 38 analyzes the optical image to specify the joint part being imaged. As a result, it is possible to confirm whether or not imaging is performed according to the imaging order of the preset information.

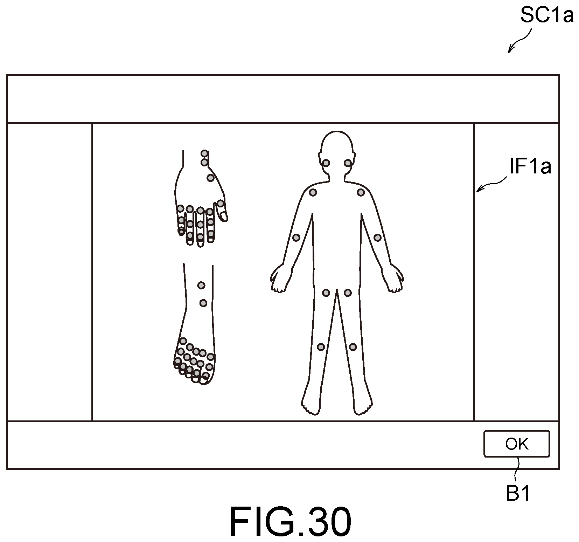

In the ultrasonic diagnostic apparatus 1 according to the first and second embodiments and Modifications 1 to 7 described above, in the preset information, not only the imaging order of the joint part of the finger but also the imaging order of the entire body of the subject may be set. FIG. 30 is a diagram illustrating an example of the preset information setting screen, and is a diagram corresponding to FIG. 2. As illustrated in FIG. 30, preset information IF1a and a first button B1 are displayed on a preset information setting screen SC1a. The preset information IF1a includes an imaging order. Specifically, the preset information IF1a includes an imaging order for a plurality of joint parts of the subject P. That is, the imaging order is set for the plurality of joint parts of the subject P. In the example illustrated in FIG. 2, in the preset information IF1a, the imaging order for the joint parts of the entire body among the plurality of joint parts is set.

On the result display screen SC5 in the ultrasonic diagnostic apparatus 1 according to the first and second embodiments and Modifications 1 to 7 described above, when the inspector performs the input operation of selecting any of the plurality of joint parts in both the hands via the input device 50 and the plurality of pieces of ultrasonic image data is stored in the selected joint part, the display control function 382 may accept the input operation of selecting one ultrasonic image data from the plurality of pieces of ultrasonic image data from the user, may acquire ultrasonic image data having a largest number of blood flow signals, or may acquire ultrasonic image data having a largest area of the synovial region.

The indicator needle N1 in the indicator which is the information IF2 capable of identifying the category corresponding to the index value in the ultrasonic diagnostic apparatus 1 according to the first and second embodiments and Modifications 1 to 7 indicates the category corresponding to the index value, but the present disclosure is not limited thereto. The indicator needle N1 may indicate the index value. That is, the indicator needle N1 may rotate by an amount corresponding to the index value. In the example illustrated in FIG. 3, for example, when the index value is 60, the indicator needle N1 may be rotated such that the indicator needle N1 indicates 60, that is, a distal end of the indicator needle N1 moves to a position of 60.

In the detection processing executed in the ultrasonic diagnostic apparatus 1 according to the first and second embodiments and Modifications 1 to 7 described above, each of the plurality of pieces of processed image data is input to the trained model LM to detect the synovial region in each of the plurality of pieces of processed image data and select one synovial region, but the present disclosure is not limited thereto. In the ultrasonic diagnostic apparatus 1 according to the first and second embodiments and Modifications 1 to 7 described above, the synovial region in each of the plurality of pieces of processed image data may be detected by inputting each of the plurality of pieces of processed image data, and the synovial region of the ultrasonic image data may be detected by calculating an intersection or a union between the synovial regions of the plurality of pieces of detected processed image data.

The index value calculation function 387 in the ultrasonic diagnostic apparatus 1 according to the first and second embodiments and Modifications 1 to 7 described above can calculate the index value by inputting the image data in which the power Doppler image data is superimposed on the B mode image data, to the trained model that receives, as inputs, the image data in which the power Doppler image data is superimposed on the B mode image data, receives, as inputs, the image data in which the power Doppler image data is superimposed on the B mode image data, and outputs the index value.

Note that the word "processor" used in above descriptions means circuits such as, for example, a Central Processing Unit (CPU), a Graphics Processing Unit (GPU), an Application Specific Integrated Circuit (ASIC), a programmable logic device (for example, a Simple Programmable Logic Apparatus (SPLD), a Complex Programmable Logic Apparatus (CPLD), and a Field Programmable Gate Array (FPGA)). The processor executes functions by reading and executing programs stored in the storage 37,501. Note that programs may be configured to be directly integrated in the processor instead of being storing in the storage 37,501. In this case, the processor realizes functions by reading and executing programs stored in the circuitry. Note that the processor is not limited to the case arranged as a single processor circuit, but may be configured as a single processor by combining a plurality of independent circuits to realize functions. Furthermore, a plurality of component elements in Fig.1 may be integrated into one processor to realize the functions.

According to at least one embodiment described above, the degree of dependence on the inspector in the diagnosis of rheumatism can be reduced.

While certain embodiments have been described, these embodiments have been presented by way of example only and are not intended to limit the scope of the inventions. The embodiments may be in a variety of other forms. Furthermore, various omissions, substitutions and changes may be made without departing from the spirit of the inventions. The embodiments and their modifications are included in the scope and the subject matter of the invention, and at the same time included in the scope of the claimed inventions.

## Claims

1. An ultrasonic diagnostic apparatus (1) comprising
processing circuitry (38) configured to:
acquire ultrasonic image data indicating a target part of a subject;
acquire first evaluation information for evaluating a state of rheumatism in the target part indicated by the ultrasonic image data;
acquire second evaluation information for evaluating the state of rheumatism in the target part;
calculate an index value indicating the state of rheumatism based on information including at least the first evaluation information and the second evaluation information; and
display information capable of identifying a category corresponding to the index value among a plurality of categories indicating a degree of the state of rheumatism.

2. The ultrasonic diagnostic apparatus of claim 1, wherein pieces of visual information different from each other are assigned to the plurality of categories.

3. The ultrasonic diagnostic apparatus of claim 2,
wherein the first evaluation information is a synovial region (R1), and
wherein the visual information is given to at least a part of the synovial region.

4. The ultrasonic diagnostic apparatus of either of claims 1 or 2,
wherein the first evaluation information is a synovial region (R1), and
wherein the processing circuitry (38) is further configured to detect the synovial region in each of a plurality of pieces of processed image data by inputting, to a trained model, each of the plurality of pieces of processed image data from each other obtained by executing image processing on the ultrasonic image data, the trained model being configured to output the synovial region (R1) in the ultrasonic image data in response to inputting the ultrasonic image data into the trained model.

5. The ultrasonic diagnostic apparatus of any preceding claim,
wherein an imaging order is set for a plurality of target parts of the subject, and
wherein the processing circuitry (38) is further configured to register the information capable of identifying the category for each of the plurality of target parts.

6. The ultrasonic diagnostic apparatus of claim 5,
wherein the processing circuitry (38) is further configured to:
register preset information including the imaging order as a protocol; and
display information based on the imaging order during execution of the protocol.

7. The ultrasonic diagnostic apparatus of claim 6, wherein the processing circuitry (38) is further configured to select a body mark corresponding to the imaging order based on the preset information.

8. The ultrasonic diagnostic apparatus of claim 5, wherein the processing circuitry (38) is further configured to display information regarding a target part of the subject to be imaged next, as the information based on the imaging order.

9. The ultrasonic diagnostic apparatus of claim 5, wherein the processing circuitry (38) is further configured to display information indicating that imaging is completed, as the information based on the imaging order.

10. The ultrasonic diagnostic apparatus of claim 1,
wherein the first evaluation information is a synovial region (R1), and
wherein the processing circuitry (38) is further configured to calculate, as the index value, a ratio of the number of pixels of the blood flow signal in the synovial region (R1) to the number of pixels of the synovial region (R1) based on the number of pixels of the synovial region (R1) and the number of pixels of the blood flow signal in the synovial region (R1).

11. A medical image processing apparatus comprising
processing circuitry configured to:
acquire ultrasonic image data indicating a target part of a subject;
acquire first evaluation information for evaluating a state of rheumatism in the target part indicated by the ultrasonic image data;
acquire second evaluation information for evaluating the state of rheumatism in the target part;
calculate an index value indicating the state of rheumatism based on information including at least the first evaluation information and the second evaluation information; and
display information capable of identifying a category corresponding to the index value among a plurality of categories indicating a degree of the state of rheumatism.

12. A medical image processing method comprising:
acquiring ultrasonic image data indicating a target part of a subject;
acquiring first evaluation information for evaluating a state of rheumatism in the target part indicated by the ultrasonic image data;
acquiring second evaluation information for evaluating the state of rheumatism in the target part;
calculating an index value indicating the state of rheumatism based on information including at least the first evaluation information and the second evaluation information; and
displaying information capable of identifying a category corresponding to the index value among a plurality of categories indicating a degree of the state of rheumatism.

13. A program causing a computer to:
acquire ultrasonic image data indicating a target part of a subject;
acquire first evaluation information for evaluating a state of rheumatism in the target part indicated by the ultrasonic image data;
acquire second evaluation information for evaluating the state of rheumatism in the target part;
calculate an index value indicating the state of rheumatism based on information including at least the first evaluation information and the second evaluation information; and
display information capable of identifying a category corresponding to the index value among a plurality of categories indicating a degree of the state of rheumatism.
